# EUROPEAN PATENT SPECIFICATION

(11) **EP 3 773 840 B1**
(45) Date of publication and mention of the grant of the patent: **06.11.2024**
(21) Application number: 19780574.0
(22) Date of filing: 02.04.2019
(51) Int. Cl.: A61M 16/00, A61M 16/10, A61M 16/20, A61M 15/00, A61K 9/00

(54) **HANDHELD DIGITAL NEBULIZER DEVICE AND METHODS OF USE**
DIGITALER HANDVERNEBLER UND VERWENDUNGSVERFAHREN
DISPOSITIF NÉBULISEUR NUMÉRIQUE PORTATIF ET PROCÉDÉS D'UTILISATION

(30) Priority: 02.04.2018 US 201862651706 P
(43) Date of publication of application: 17.02.2021
(73) Proprietor: Pneuma Respiratory, Inc., Boone, NC 28607 (US)
(72) Inventor: HEBRANK, John H., Boone, North Carolina 28607 (US); HUNTER, Charles Eric, Boone, North Carolina 28607 (US)
(74) Representative: Greaves Brewster LLP
(86) International application number: PCT/US2019/025321
(87) International publication number: WO 2019/195239

(56) References cited:
- WO-A1-2017/192767
- WO-A1-98/48873
- AU-A1- 2012 258 488
- US-A- 5 884 620
- US-A- 6 071 498
- US-A1- 2003 140 921
- US-A1- 2015 018 694
- US-A1- 2015 196 060
- US-A1- 2015 352 301
- US-A1- 2017 319 797

## Description

### RELATED APPLICATIONS

The present application claims benefit under 35 U.S.C. § 119 of U.S. Provisional Patent Application No. 62/651,706, filed April 2, 2018, entitled "HANDHELD DIGITAL NEBULIZER DEVICE AND METHODS OF USE".

### FIELD OF THE INVENTION

This disclosure relates to nebulizer devices and more specifically to nebulizer devices for the delivery of fluids to the pulmonary system.

### BACKGROUND OF THE INVENTION

The use of aerosol generating devices such as nebulizers for the treatment of a variety of respiratory diseases is an area of large interest. Inhalation provides for the delivery of aerosolized drugs to treat asthma, COPD and site-specific conditions, with reduced systemic adverse effects.

Nebulizers use various methods to create droplets, so the efficacy can vary greatly. Common commercially available nebulizers deliver medication during both inhalation and exhalation, which results in approximately 50% of the dose being wasted. As such, there is significant waste of the medication and significant issues verifying the dosage of medication actually received by the patient. A major challenge is providing a nebulizer device that delivers an accurate, consistent, and verifiable dose, with a droplet size that is suitable for successful delivery of medication to the targeted lung passageways.

Blockage of ejector apertures in aerosol generating devices by deposited drug residue and surface condensation is also a problem for existing vibrating mesh or aperture plate nebulizers. In these systems, in order to prevent a buildup of drug onto mesh aperture surfaces, manufacturers require repeated washing and cleaning, as well as disinfection after a single use in order to prevent possible microbiological contamination. Other challenges include delivery of viscous drugs and suspensions that can clog the apertures or pores and lead to inefficiency or inaccurate drug delivery to patients or render the device inoperable. Also, the use of detergents or other cleaning or sterilizing fluids may damage the ejector mechanism or other parts of the nebulizer and lead to uncertainty as to the ability of the device to deliver a correct dose to the patient or state of performance of the device.

Accordingly, there is a need for a nebulizer device that delivers droplets of a suitable size range that avoids surface fluid deposition and blockage of apertures, with a dose that is verifiable, and provides feedback regarding correct and consistent usage of the nebulizer to patient and professional such as physician, pharmacist or therapist.

### SUMMARY OF THE INVENTION

In certain embodiments, the disclosure relates to a breath actuated, handheld nebulizer device for delivering fluid as an ejected stream of droplets to the pulmonary system of a subject. In certain embodiments, the handheld nebulizer device includes an exhalation valve of a suitable size, shape, and configuration so as to allow a subject user to exhale during use without causing pressure back pressure through the device. In certain embodiments, the handheld nebulizer device of the disclosure is configured in an in-line orientation in that the housing, its internal components, and various device components (e.g., the mouthpiece, air inlet flow element, etc.) are orientated in a substantially in-line or parallel configuration (e.g., along the airflow path) so as to form a small, hand-held device.

In certain embodiments, the handheld nebulizer device may include: a housing including an exhalation valve; a mouthpiece positioned at the airflow exit side of the housing; a reservoir disposed within or in fluid communication with the housing for receiving a volume of fluid; an ejector mechanism in fluid communication with the reservoir, the ejector mechanism comprising a piezoelectric actuator and an aperture plate, the aperture plate having a plurality of openings formed through its thickness and the piezoelectric actuator operable to oscillate the aperture plate at a frequency to thereby generate an ejected stream of droplets, at least one differential pressure sensor positioned within the housing; the at least one differential pressure sensor configured to activate the ejector mechanism upon sensing a pre-determined pressure change within the mouthpiece to thereby generate an ejected stream of droplets; the ejector mechanism configured to generate the ejected stream of droplets wherein at least about 50% of the droplets have an average ejected droplet diameter of less than about 6 microns, such that at least about 50% of the mass of the ejected stream of droplets is delivered in a respirable range to the pulmonary system of a subject during use.

In some aspects, the handheld nebulizer device further includes an air inlet flow element positioned in the airflow at the airflow entrance of the device and configured to facilitate non-turbulent (i.e., laminar and/or transitional) airflow across the exit side of aperture plate and to provide sufficient airflow to ensure that the ejected stream of droplets flows through the handheld nebulizer device during use. In some embodiments, the air inlet flow element may be positioned within the mouthpiece.

In certain embodiments, the housing and ejector mechanism are oriented such that the exit side of the aperture plate is perpendicular to the direction of airflow and the stream of droplets is ejected in parallel to the direction of airflow. In other embodiments, the housing and ejector mechanism are oriented such that the exit side of the aperture plate is parallel to the direction of airflow and the stream of droplets is ejected substantially perpendicularly to the direction of airflow such that the ejected stream of droplets is directed through the housing at an approximate 90 degree change of trajectory prior to expulsion from the housing.

In certain aspects, the handheld nebulizer device further includes a surface tension plate between the aperture plate and the reservoir, wherein the surface tension plate is configured to increase contact between the volume of fluid and the aperture plate. In other aspects, the ejector mechanism and the surface tension plate are configured in parallel orientation. In yet other aspects, the surface tension plate is located within 2 mm of the aperture plate so as to create sufficient hydrostatic force to provide capillary flow between the surface tension plate and the aperture plate.

In yet other aspects, the aperture plate of the handheld nebulizer device comprises a domed shape. In other aspects, the aperture plate may be formed of a metal, e.g., stainless steel, nickel, cobalt, titanium, iridium, platinum, or palladium or alloys thereof. Alternatively, the aperture plate can be formed of suitable material, including other metals or polymers. In certain embodiments, the aperture plate is comprised of, e.g., poly ether ether ketone (PEEK), polyimide, polyetherimide, polyvinylidine fluoride (PVDF), ultra-high molecular weight polyethylene (UHMWPE), nickel, nickel-cobalt, palladium, nickel-palladium, platinum, or other suitable metal alloys, and combinations thereof. In other aspects, one or more of the plurality of openings of the aperture plate have different cross-sectional shapes or diameters to thereby provide ejected droplets having different average ejected droplet diameters.

In yet other aspects, the reservoir of the handheld nebulizer device is removably coupled with the housing. In other aspects, the reservoir of the handheld nebulizer device is coupled to the ejector mechanism to form a combination reservoir/ejector mechanism module, and the combination reservoir/ejector mechanism module is removably coupled with the housing.

In other aspects, the handheld nebulizer device may further include a wireless communication module. In some aspects, the wireless communication module is a Wi-Fi, cellular, or Bluetooth^{®} transmitter.

In yet other aspects, the handheld nebulizer device may further include one or more sensors selected from an infer-red transmitter, a photodetector, an additional pressure sensor, and combinations thereof.

In one aspect, the disclosure relates to a method for generating and delivering a fluid as an ejected stream of droplets to the pulmonary system of a subject in a respirable range. The method may comprise: (a) generating an ejected stream of droplets via a breath actuated handheld nebulizer device of the disclosure, wherein at least about 50% of the ejected stream of droplets have an average ejected droplet diameter of less than about 6 µm; and (b) delivering the ejected stream of droplets to the pulmonary system of the subject such that at least about 50% of the mass of the ejected stream of droplets is delivered in a respirable range to the pulmonary system of a subject during use.

While multiple embodiments are disclosed, still other embodiments of the present disclosure will become apparent to those skilled in the art from the following detailed description, which shows and describes illustrative embodiments of the disclosure. As will be realized, the invention is capable of modifications in various aspects, all without departing from the scope of the present disclosure. Accordingly, the detailed descriptions are to be regarded as illustrative in nature and not restrictive.

### BRIEF DESCRIPTION OF THE DRAWINGS

**FIGS. 1A****-1B** illustrate perspective views of an exemplary handheld nebulizer device, in accordance with embodiments of the disclosure.
**FIGS. 1C****-1D** illustrate an exemplary mouthpiece having an exhalation valve, in accordance with embodiments of the disclosure.
**FIG. 2** is an exploded view of a handheld nebulizer device of **FIG. 1A****-1B,** in accordance with embodiments of the disclosure.
**FIG. 3A-1** is a partial perspective view of a base unit of a handheld nebulizer device of **FIG. 1A****-1B,** in accordance with embodiments of the disclosure.

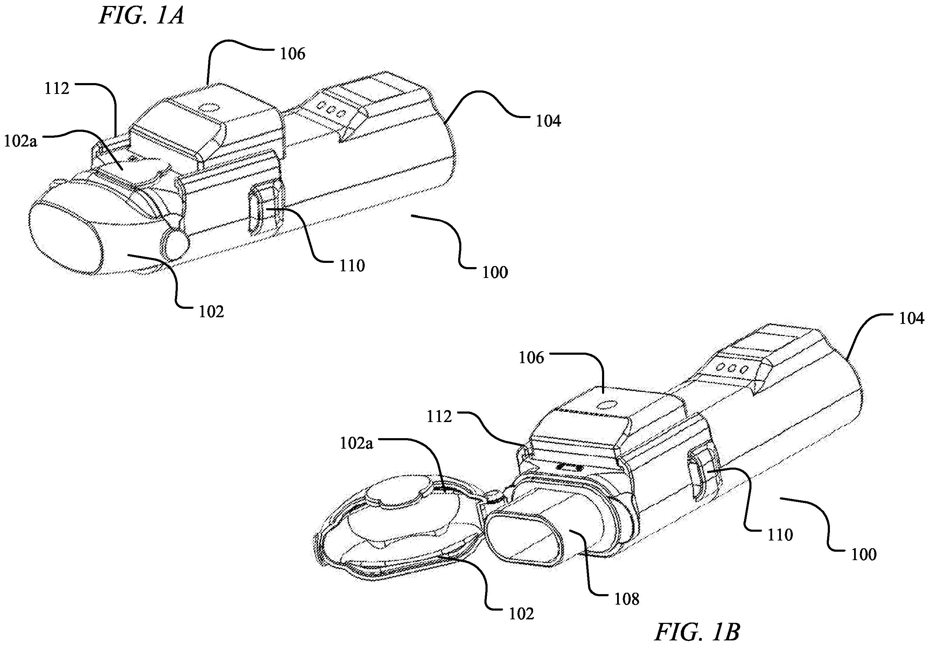

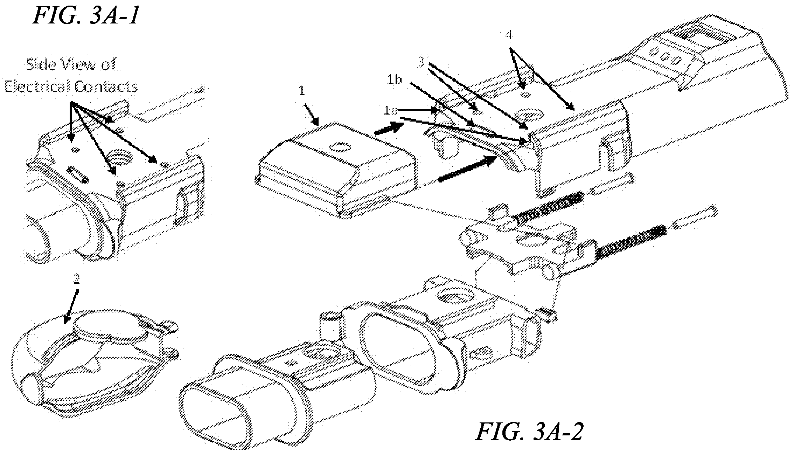

**FIG. 3A-2** is an exploded view of a handheld nebulizer device of **FIG. 1A-****1B,** in accordance with embodiments of the disclosure.

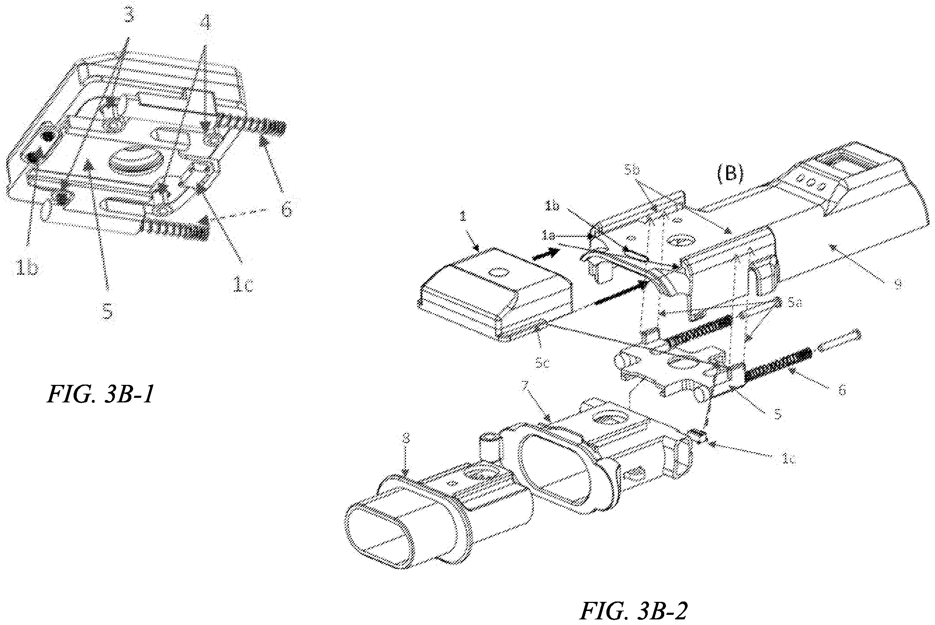

**FIG. 3B-1** is a bottom perspective view of a drug delivery ampoule of a handheld nebulizer device of **FIG. 1A****-1B,** in accordance with embodiments of the disclosure.
**FIG. 3B-2** is an exploded view of a handheld nebulizer device of **FIG. 1A****-1B,** in accordance with embodiments of the disclosure.
**FIGS. 3C-1**, **3C-2**, and **3C-3** are cross section perspective views of a handheld nebulizer device of **FIG. 1A****-1B,** in accordance with embodiments of the disclosure.

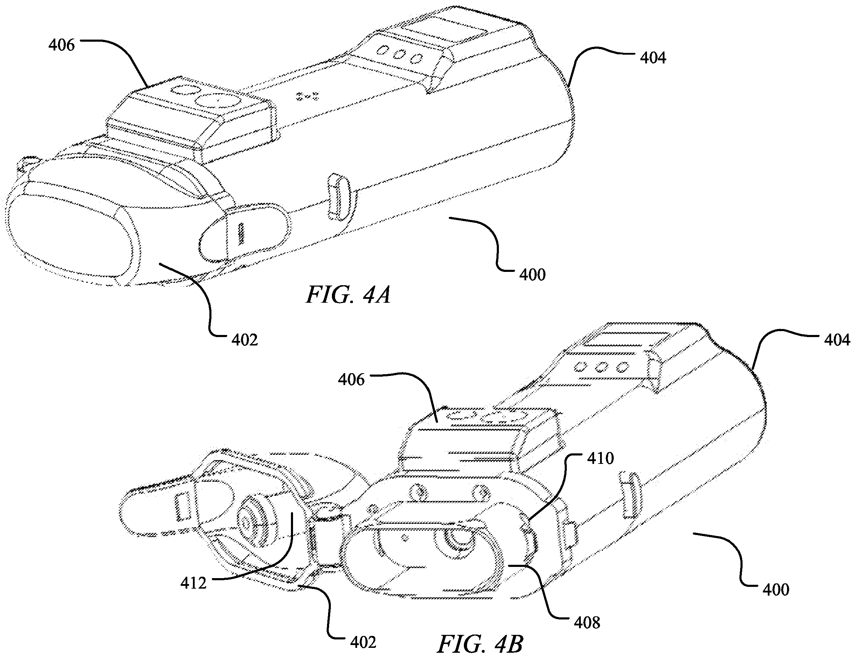

**FIGS. 4A****-4B** illustrate perspective views of another exemplary handheld nebulizer device, in accordance with embodiments of the disclosure.

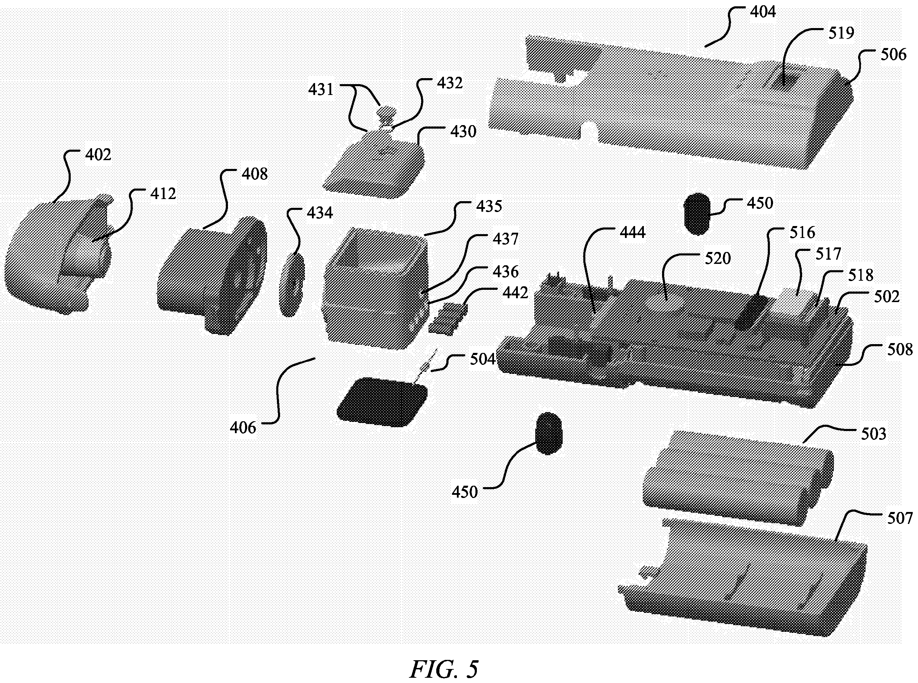

**FIG. 5** is an exploded view of a handheld nebulizer device of **FIG. 4A****-4B,** in accordance with embodiments of the disclosure.

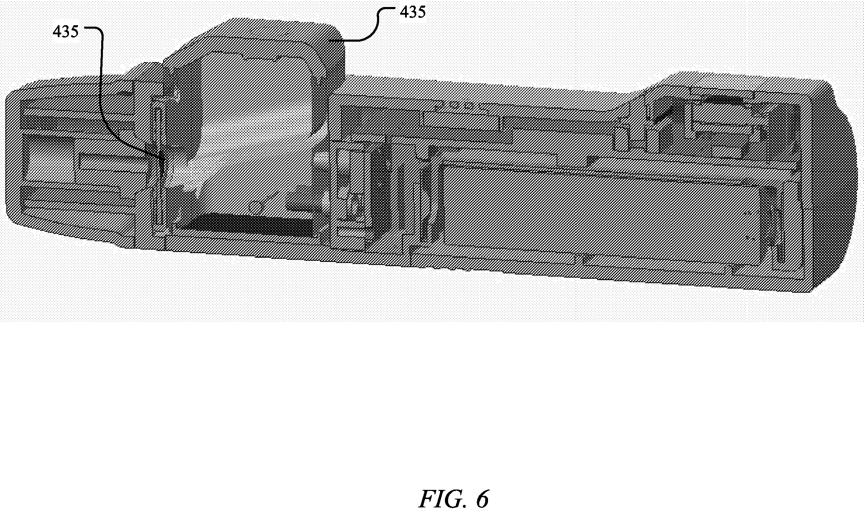

**FIG. 6** is a cross section perspective view of a handheld nebulizer device of **FIG. 4A****-4B,** in accordance with embodiments of the disclosure.

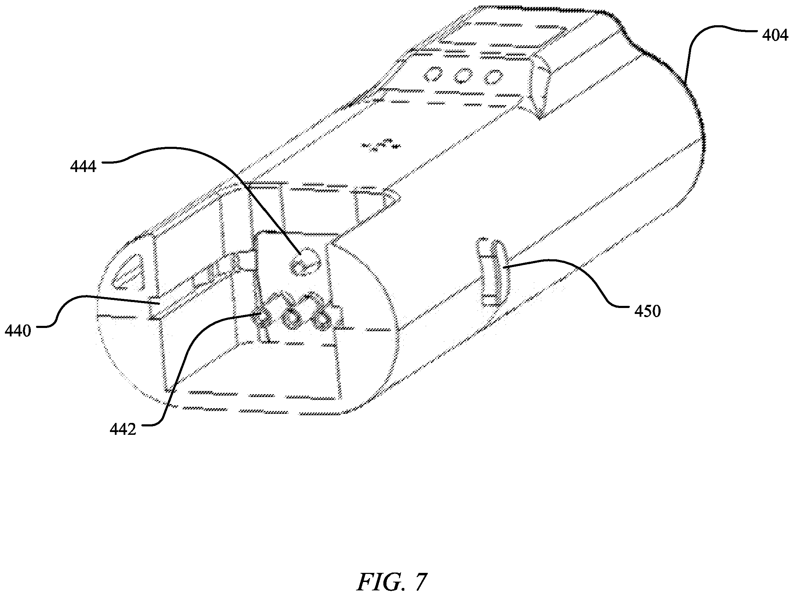

**FIG. 7** is a perspective view of a handheld nebulizer device of **FIG. 4A****-4B** without the drug delivery ampoule inserted, in accordance with embodiments of the disclosure.

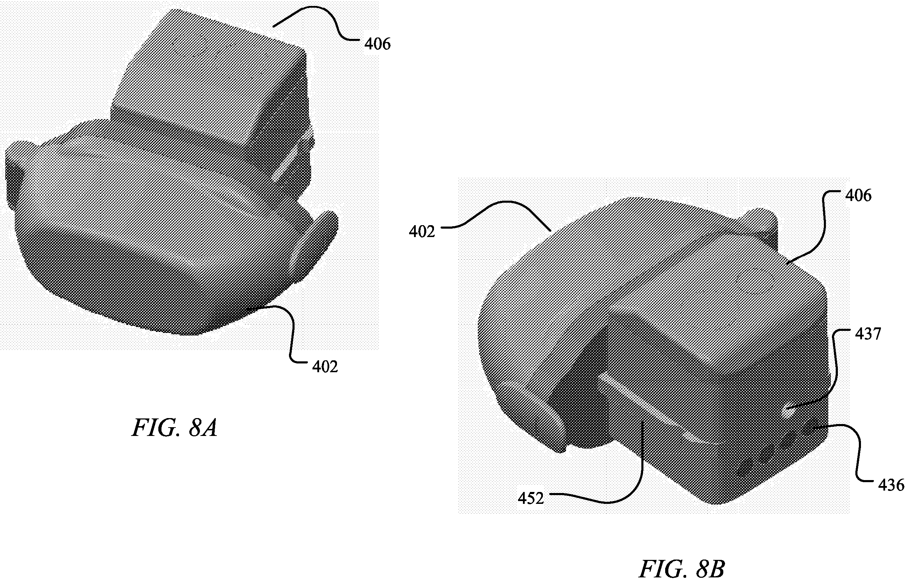

**FIGS. 8A****-8B** are perspective views of a drug delivery ampoule and mouthpiece cover, showing a front view **(****FIG. 8A****)** and back view **(****FIG. 8B****),** in accordance with embodiments of the disclosure.

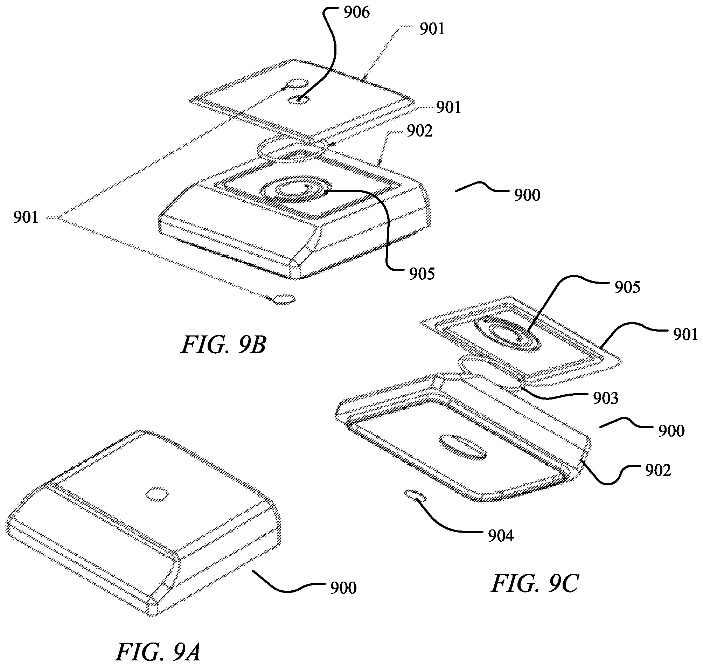

**FIGS. 9A****-9D** show alternative drug delivery ampoules. **FIG. 9A** shows a perspective view of a first embodiment of a drug delivery ampoule, with **FIG. 9B** showing a top exploded view and **FIG. 9C** showing a bottom exploded view of the ampoule of **FIG. 9A.** FIG. 9A illustrates a cross-section of an alternative embodiment of drug delivery ampoule, in accordance with embodiments of the disclosure.

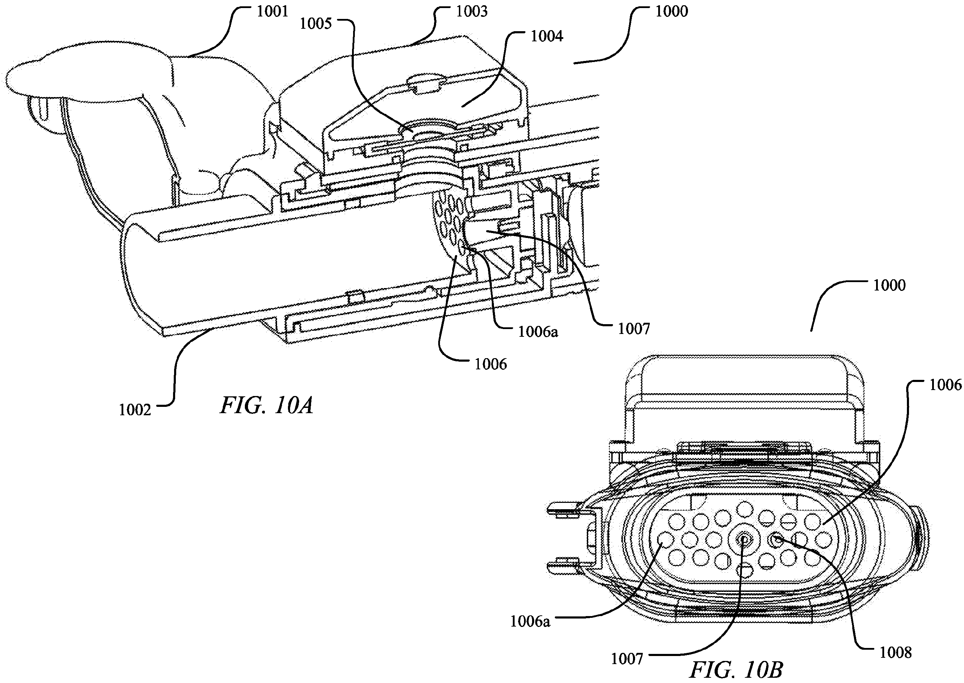

**FIG. 10A** is a partial cross section perspective view of a handheld nebulizer device of **FIG. 1A****-1B** comprising a drug delivery ampoule, mouthpiece including an air inlet flow element, and mouthpiece cover, in accordance with an embodiment of the disclosure.
**FIG. 10B** is a front view of a handheld nebulizer device of **FIG. 1A****-1B** comprising a drug delivery ampoule and mouthpiece including an air inlet flow element, in accordance with an embodiment of the disclosure.

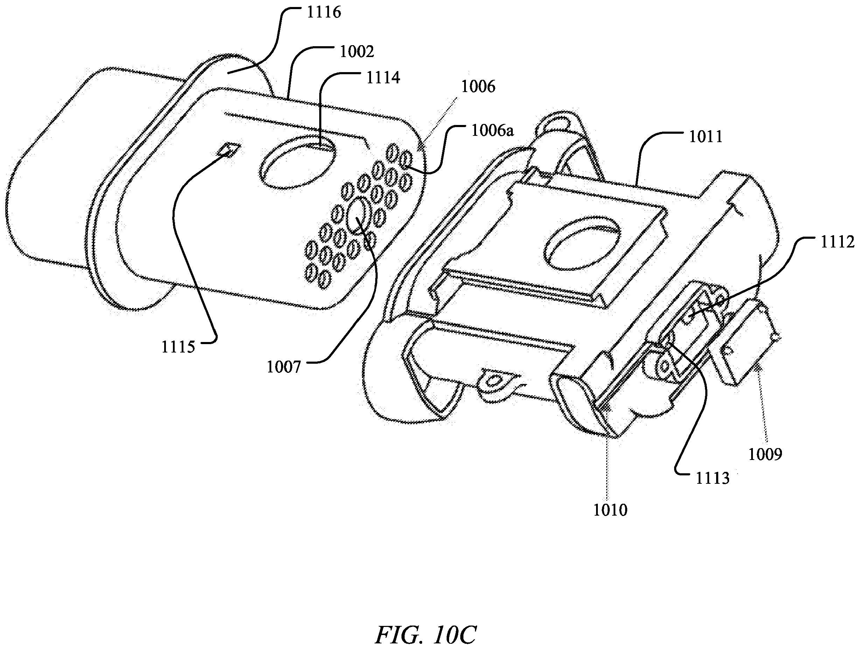

**FIG. 10C** is an exploded view of components of a handheld nebulizer device of **FIG. 1A****-1B** including a mouthpiece and internal housing, in accordance with an embodiment of the disclosure.

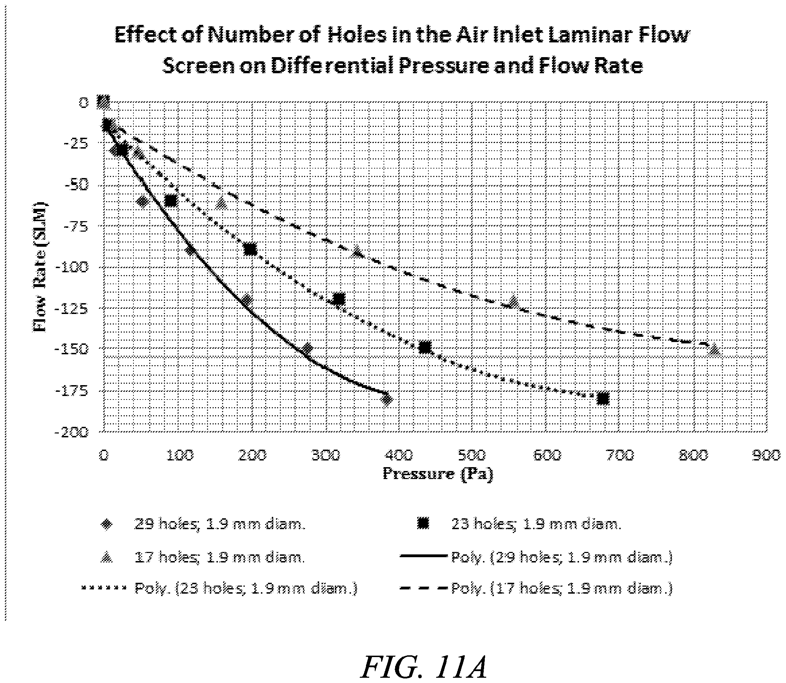

**FIG. 11A** is a plot of the differential pressure as a function of flow rates through exemplary air inlet flow elements as a function of number of holes, in accordance with an embodiment of the disclosure.

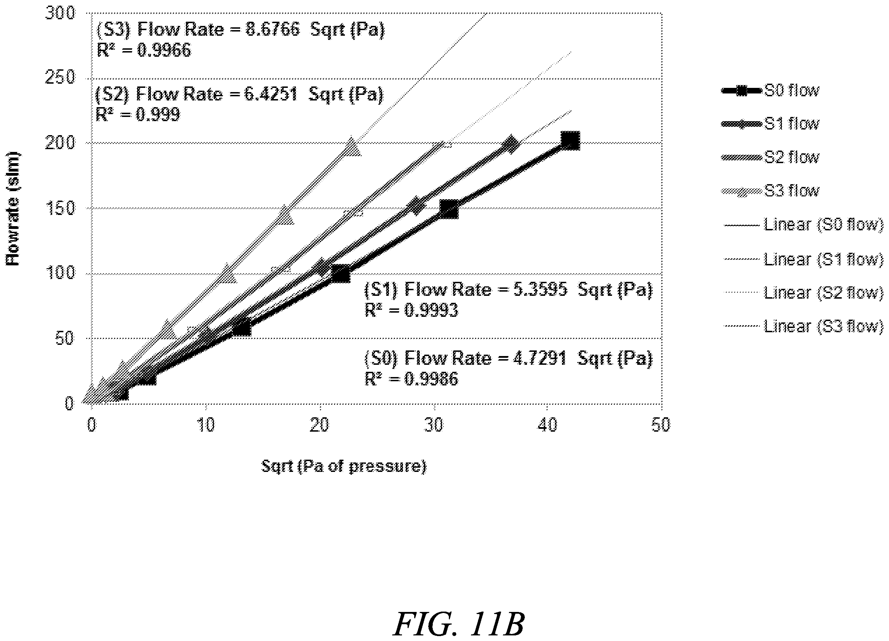

**FIG. 11B** is a plot of the differential pressure as a function of flow rates through exemplary air inlet flow elements as a function of screen hole size and number of holes set at a constant, 17 holes, in accordance with an embodiment of the disclosure..

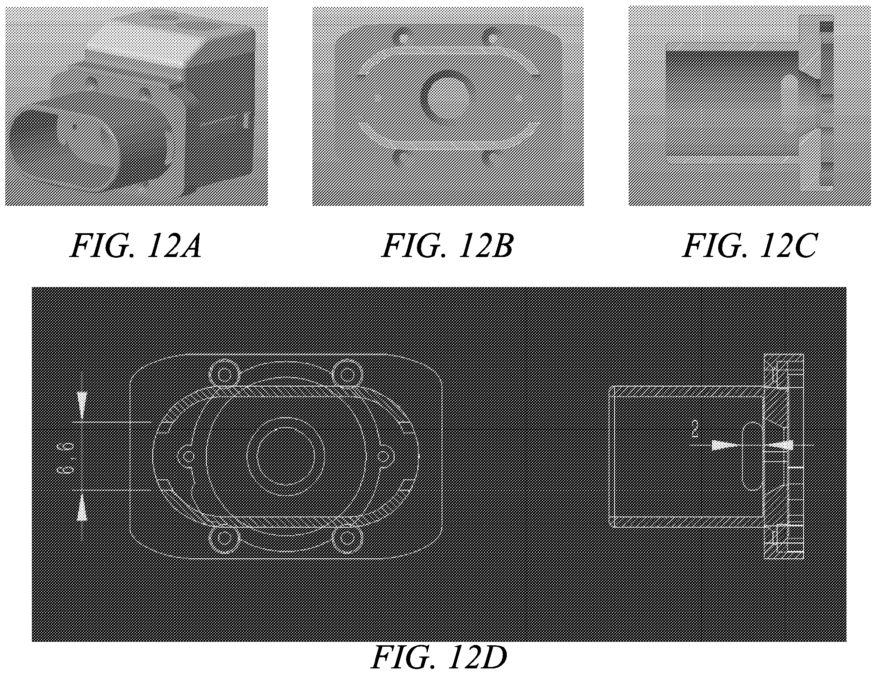

**FIG. 12A** shows an exemplary drug delivery ampoule with a mouthpiece interfaced at the airflow exit side of the device, in accordance with an embodiment of the disclosure. **FIG. 12B** shows a front cross-section and **FIG. 12C** shows a side cross-section, with **FIG. 12D** showing the same views with exemplary dimensions.

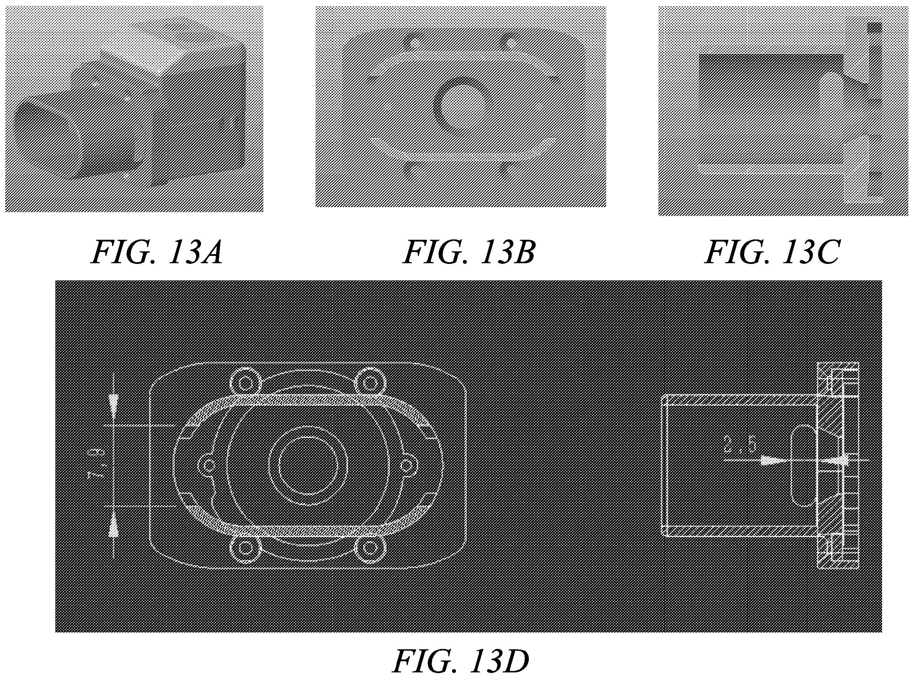

**FIG. 13A** shows an alternative drug delivery ampoule with a mouthpiece interfaced at the airflow exit side of the device, in accordance with an embodiment of the disclosure. **FIG. 13B** shows a front cross-section and **FIG. 13C** shows a side cross-section, with **FIG. 13D** showing the same views with exemplary dimensions.

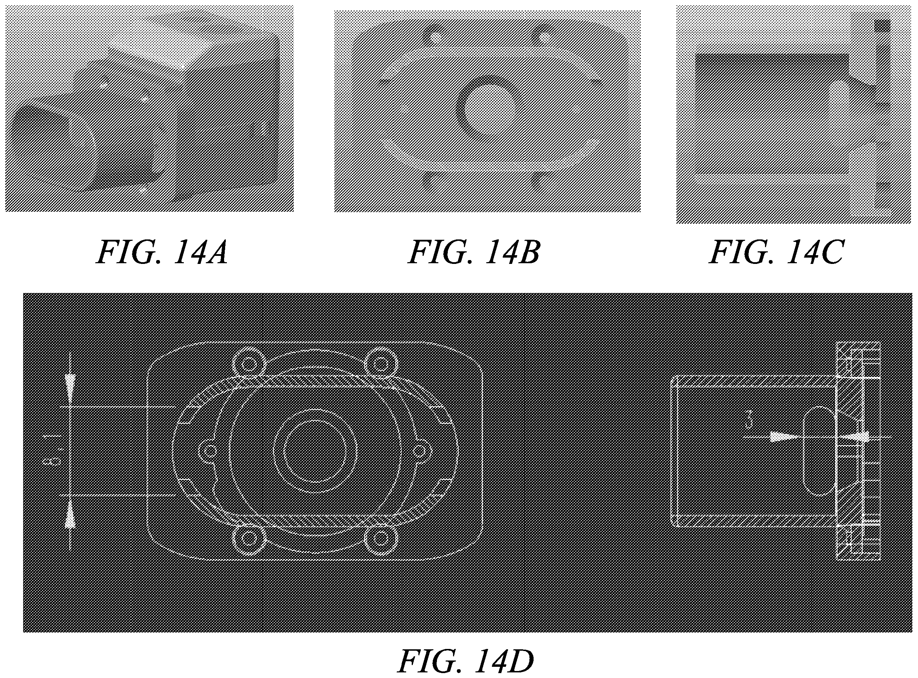

**FIG. 14A** shows an alternative drug delivery ampoule with a mouthpiece interfaced at the airflow exit side of the device, in accordance with an embodiment of the disclosure. **FIG. 14B** shows a front cross-section and **FIG. 14C** shows a side cross-section, with **FIG. 14D** showing the same views with exemplary dimensions.

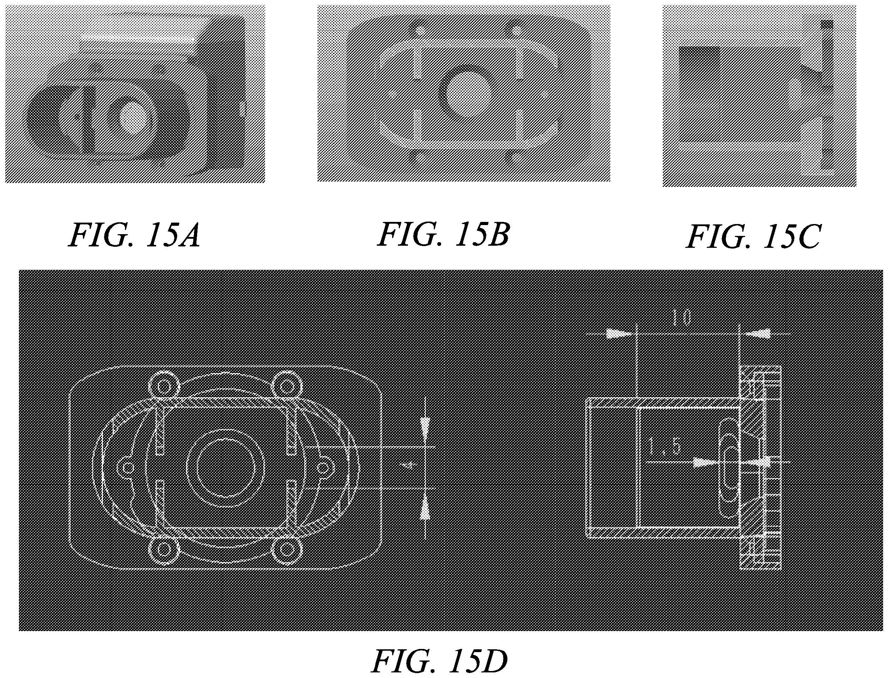

**FIG. 15A** shows an exemplary drug delivery ampoule with a mouthpiece interfaced at the airflow exit side of the device, in accordance with an embodiment of the disclosure. The mouthpiece includes two airflow entrances on the exterior sides of the mouthpiece, and two interior baffles with additional airflow entrances to provide resistance and modeling of airflow. **FIG. 15B** shows a front cross-section and **FIG. 15C** shows a side cross-section, with **FIG. 15D** showing the same views with exemplary dimensions.

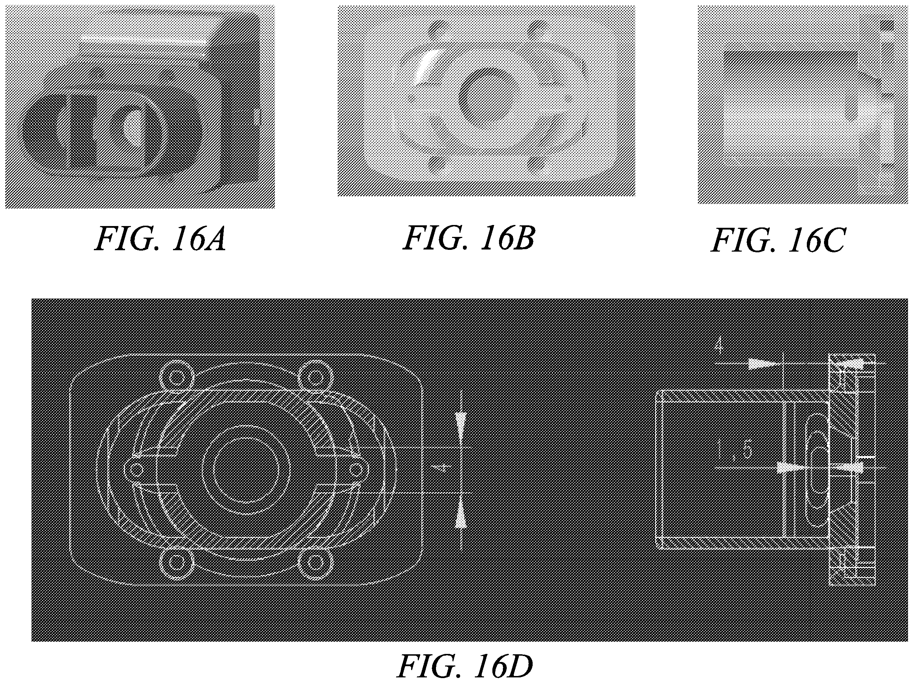

**FIG. 16A** shows an exemplary drug delivery ampoule with a mouthpiece interfaced at the airflow exit side of the device, in accordance with an embodiment of the disclosure. The mouthpiece includes two airflow entrances on the exterior sides of the mouthpiece, and two interior baffles with additional airflow entrances to provide resistance and modeling of airflow. **FIG. 16B** shows a front cross-section and **FIG. 16C** shows a side cross-section, with **FIG. 16D** showing the same views with exemplary dimensions.

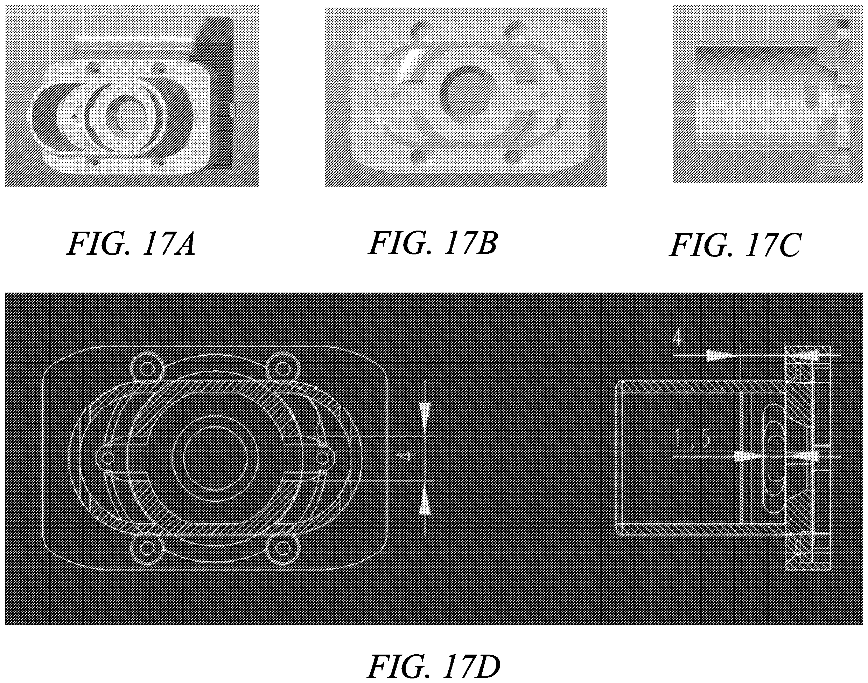

**FIG. 17A** shows an exemplary drug delivery ampoule with a mouthpiece interfaced at the airflow exit side of the device, in accordance with an embodiment of the disclosure. The mouthpiece includes two airflow entrances on the exterior sides of the mouthpiece, and a substantially concentric baffle (two arcs that form a circle with the top and bottom of the mouthpiece) with two additional airflow entrances to provide resistance and modeling of airflow. **FIG. 17B** shows a front cross-section and **FIG. 17C** shows a side cross-section, with **FIG. 17D** showing the same views with exemplary dimensions.

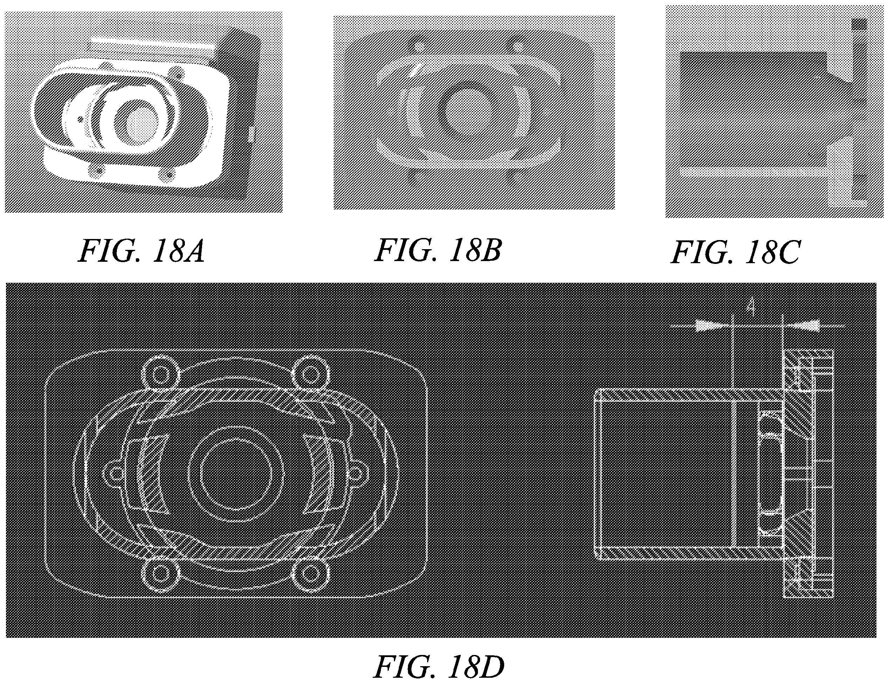

**FIG. 18A** shows an exemplary drug delivery ampoule with a mouthpiece interfaced at the airflow exit side of the device, in accordance with an embodiment of the disclosure. The mouthpiece includes two airflow entrances on the exterior sides of the mouthpiece, and a substantially concentric baffle (two arcs that form a circle with the top and bottom of the mouthpiece) with four airflow entrances to provide resistance and modeling of airflow. **FIG. 18B** shows a front cross-section and **FIG. 18C** shows a side cross-section, with **FIG. 18D** showing the same views with exemplary dimensions.

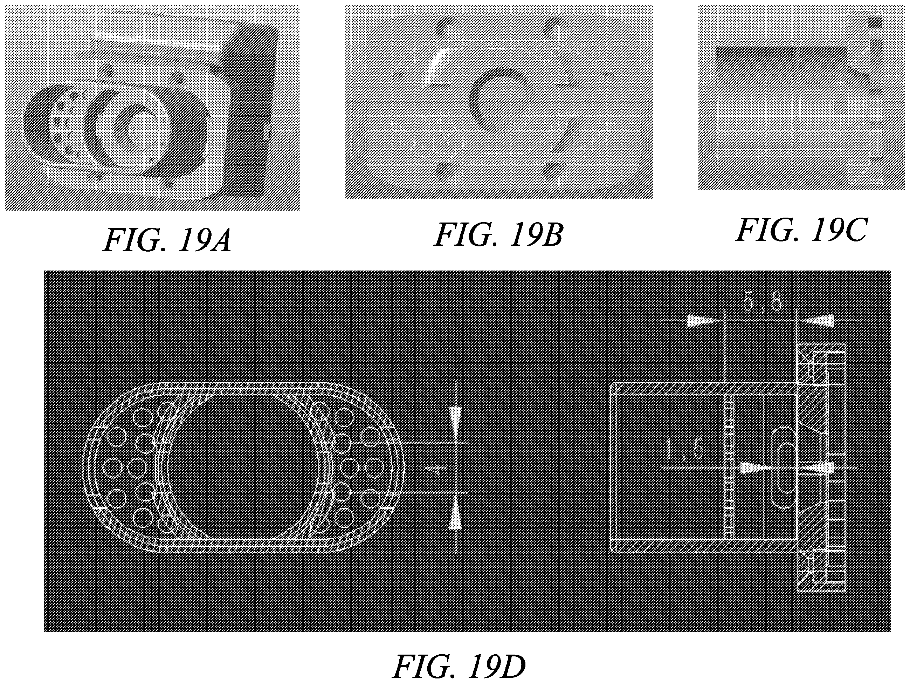

**FIG. 19A** shows an exemplary drug delivery ampoule with a mouthpiece interfaced at the airflow exit side of the device, in accordance with an embodiment of the disclosure. The mouthpiece includes two airflow entrances on the exterior sides of the mouthpiece, and a substantially concentric baffle with two additional airflow entrances to provide resistance and modeling of airflow. In addition, the interior area of the mouthpiece between the concentric baffle and the wall of the mouthpiece includes an array element positioned above the airflow entrances to provide additional resistance and modeling to airflow. The array element is positioned in a parallel arrangement with the direction of airflow. **FIG. 19B** shows a front cross-section and FIG. 1919C shows a side cross-section, with **FIG. 19D** showing the same views with exemplary dimensions.

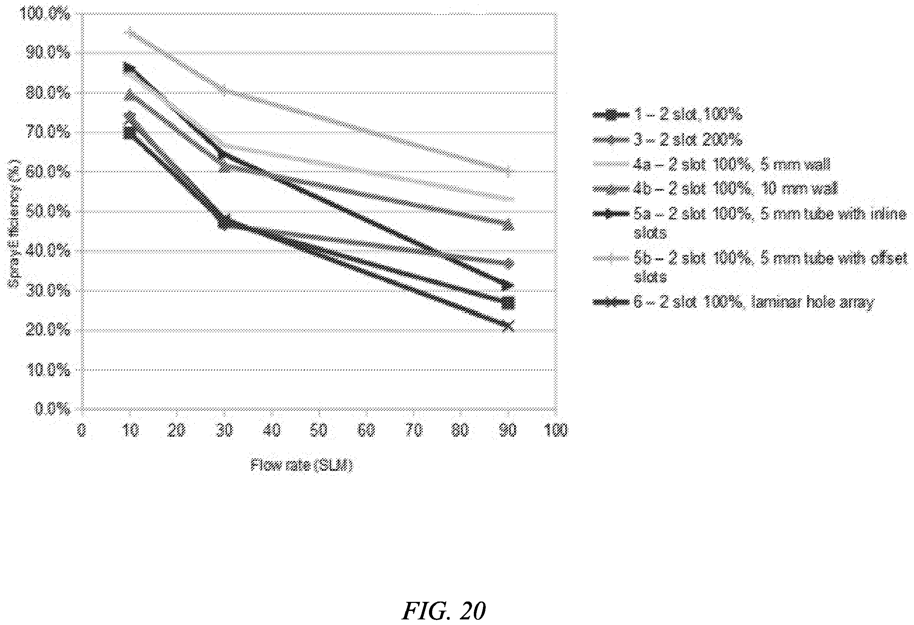

**FIG. 20** is a plot of spray efficiency as a function of flow rates through exemplary air inlet flow elements as a function of number and configuration of openings, baffles, etc., in accordance with an embodiment of the disclosure.

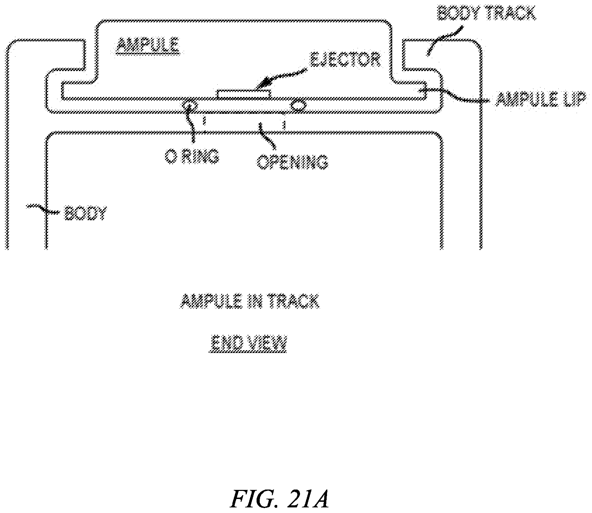

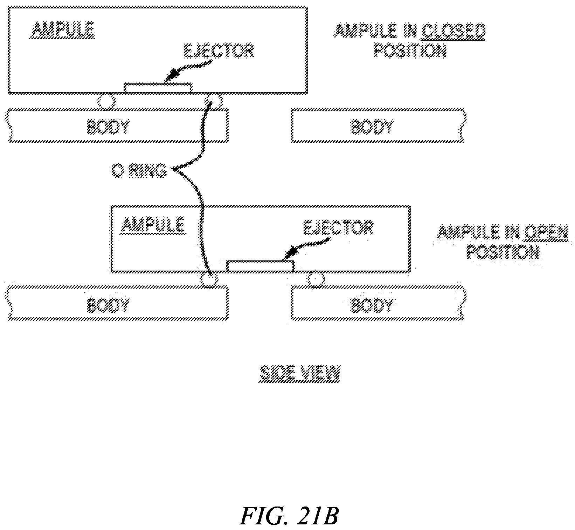

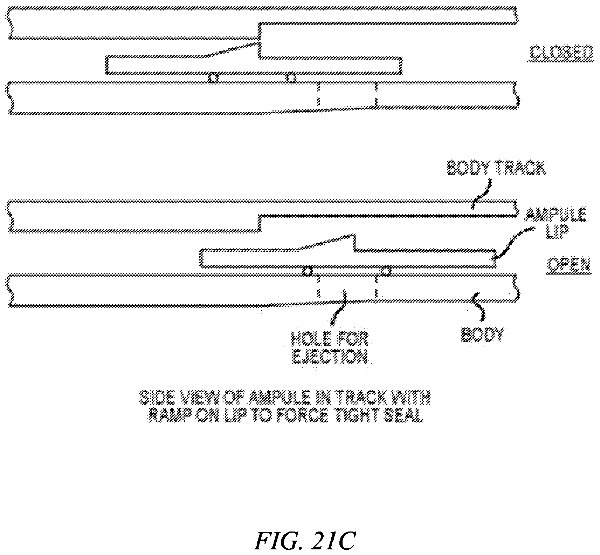

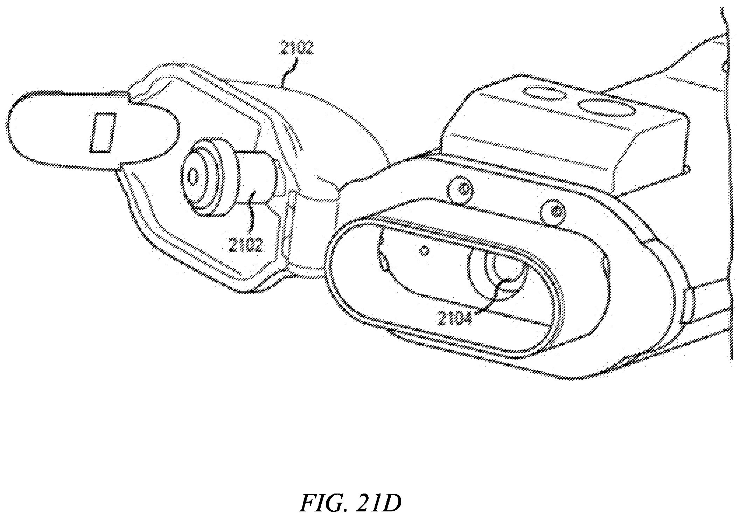

**FIGS. 21A****-21D** illustrate exemplary aperture plate seal mechanisms, in accordance with embodiments of the disclosure. **FIG. 21A** showing the ampoule in end view, **FIG. 21B** and **FIG. 21C** showing the ampoule in side view. **FIG. 21D** illustrates an alternative embodiment wherein the mouthpiece cover includes an aperture plate plug.

### DETAILED DESCRIPTION

Certain aspects of the disclosure relate to an electronic breath actuated handheld nebulizer device configured to deliver a therapeutic agent directly to the pulmonary system of a subject in need thereof, and related methods of use.

Effective delivery of medication to the deep pulmonary regions of the lungs through the alveoli, has always posed a problem, especially to children and elderly, as well as to those with the diseased state, owing to their limited lung capacity and constriction of the breathing passageways. The impact of constricted lung passageways limits deep inspiration and synchronization of the administered dose with the inspiration/expiration cycle. For optimum deposition in alveolar airways, droplets with aerodynamic diameters in the ranges of 1 to 5 µm are optimal, with droplets below about 4 µm shown to reach the alveolar region of the lungs, while larger droplets are deposited on the tongue or strike the throat and coat the bronchial passages. Smaller droplets, for example less than about 1 µm that penetrate more deeply into the lungs have a tendency to be exhaled.

Certain aspects of the disclosure relate to a fully digital platform for delivery of inhaled therapeutics, described herein as a handheld digital nebulizer device. The handheld digital nebulizer device provides substantial improvements over current nebulizer systems by improving dosing precision, dosing reliability, and delivery to the patient. In certain embodiments, the device of the disclosure includes fully integrated monitoring capabilities designed to enhance compliance and ultimately reduce disease associated morbidity.

In certain aspects of the disclosure, target diseases for which the nebulizer devices of the disclosure are particularly suited for use in the treatment and/or prevention of include asthma, Chronic Obstructive Pulmonary Disease (COPD), cystic fibrosis (CF), bronchitis, and pneumonia.

In certain aspects of the disclosure, a handheld digital nebulizer device is disclosed, which overcomes limitations of currently available nebulizer devices.

In certain aspects, the present disclosure relates to a handheld digital nebulizer device for delivery a fluid as an ejected stream of droplets to the pulmonary system of a subject and related methods of delivering safe, suitable, and repeatable dosages to the pulmonary system of a subject. The present disclosure also includes a handheld digital nebulizer device and system capable of delivering a defined volume of fluid in the form of an ejected stream of droplets such that an adequate and repeatable high percentage of the droplets are delivered into the desired location within the airways, e.g., the alveolar airways of the subject during use.

The present disclosure provides a handheld digital nebulizer device for delivery of a fluid as an ejected stream of droplets to the pulmonary system of a subject, the device comprising a housing having an exhalation valve, a reservoir for receiving a volume of fluid, and an ejector mechanism including a piezoelectric actuator and an aperture plate, wherein the ejector mechanism is configured to eject a stream of droplets having an average ejected droplet diameter of less than about 5-6 microns, preferably less than about 5 microns. As shown in further detail herein, the handheld digital nebulizer device is configured in an in-line orientation in that the housing, ejector mechanism and related electronic components are orientated in a generally in-line or parallel configuration so as to form a small, handheld device.

In certain embodiments, the housing includes an exhalation valve of a suitable size, shape, and configuration so as to allow a subject user to exhale during use without causing pressure back pressure through the device and ejector mechanism. More specifically, the exhalation valve may be configured to allow for release of pressure during exhalation to thereby minimize back pressure through the device and ejector mechanism. During use, on exhalation, the exhalation valve will open to release exhalation pressure and minimize any back pressure caused by the exhalation breath through the device and ejector mechanism.

By way of non-limiting example, the exhalation value may be configured as a flapper valve or other similar one-way valve with a very low or minimal cracking pressure (e.g., 0.02 to 0.05 psi, 0.36 psi, etc.) on exhalation during use. The exhalation valve may be sized and shaped in any suitable manner, e.g., as an oval or elongated shape that is between 5-20 mm in cross-section, e.g., 16-18 mm, 18 mm, etc. The exhalation valve may be made of any suitable material known in the art for such purposes, particularly those that are suitable for pharmaceutical uses which provide the desired cracking pressures.

In specific embodiments, the ejector mechanism is electronically breath activated by at least one differential pressure sensor located within the housing of the handheld digital nebulizer device upon sensing a pre-determined pressure change within the housing. In certain embodiments, such a pre-determined pressure change may be sensed during an inspiration cycle by a user of the device, as will be explained in further detail herein.

In certain aspects, the devices of the disclosure eliminate the need for patient / device coordination by using a differential pressure sensor to initiate the ejector mechanism in response to the onset of inhalation. The device does not require manual triggering of medication delivery. Unlike propellant driven MDIs, the droplets from the devices of the disclosure are generated having little to no intrinsic velocity from the droplet formation process and are inspired into the lungs solely by the user's incoming breath passing through the device. The droplets will ride on entrained air providing improved deposition in the lung.

In accordance with certain aspects of the disclosure, effective deposition into the lungs generally requires droplets less than about 5-6 µm in diameter. Without intending to be limited by theory, to deliver fluid to the lungs a droplet delivery device must impart a momentum that is sufficiently high to permit ejection out of the device, but sufficiently low to prevent deposition on the tongue or in the back of the throat. Droplets below approximately 5-6 µm in diameter are transported almost completely by motion of the airstream and entrained air that carry them and not by their own momentum.

In certain aspects, the present disclosure includes and provides an ejector mechanism configured to eject a stream of droplets within the respirable range of less than about 5-6 µm, preferably less than about 5 µm. The ejector mechanism is comprised of an aperture plate that is directly or indirectly coupled to a piezoelectric actuator. In certain implementations, the aperture plate may be coupled to an actuator plate that is coupled to the piezoelectric actuator. The aperture plate generally includes a plurality of openings formed through its thickness and the piezoelectric actuator directly or indirectly (e.g. via an actuator plate) oscillates the aperture plate, having fluid in contact with one surface of the aperture plate, at a frequency and voltage to generate a directed aerosol stream of droplets through the openings of the aperture plate into the lungs, as the patient inhales. In other implementations where the aperture plate is coupled to the actuator plate, the actuator plate is oscillated by the piezoelectric oscillator at a frequency and voltage to generate a directed aerosol stream or plume of aerosol droplets.

In certain aspects, the present disclosure relates to a handheld digital nebulizer device for delivering a fluid as an ejected stream of droplets to the pulmonary system of a subject. In certain aspects, the therapeutic agents may be delivered at a high dose concentration and efficacy, as compared to alternative dosing routes and standard inhalation technologies.

In certain embodiments, the handheld digital nebulizer device of the disclosure may be used to treat various diseases, disorders and conditions by delivering therapeutic agents to the pulmonary system of a subject. In this regard, the handheld digital nebulizer device may be used to deliver therapeutic agents both locally to the pulmonary system, and systemically to the body.

More specifically, the handheld digital nebulizer device may be used to deliver therapeutic agents as an ejected stream of droplets to the pulmonary system of a subject for the treatment or prevention of pulmonary diseases or disorders such as asthma, chronic obstructive pulmonary diseases (COPD) cystic fibrosis (CF), tuberculosis, chronic bronchitis, or pneumonia. In certain embodiments, the handheld digital nebulizer device may be used to deliver therapeutic agents such as COPD medications, asthma medications, or antibiotics. By way of non-limiting example, such therapeutic agents include albuterol sulfate, fenoterol, terbutaline, ipratropium bromide, glycopyrrolate, aclidinium, salmeterol, formoterol, tiotropium, umeclidinium, vilanterol, olodaterol, fluticasone proprionate, fluticasone furoate, budesonide, mometosone, cicleosonide, tobramycin, and combinations thereof.

The following table summarizes the most commonly used inhaled medications for asthma and COPD.

| **Category** | **Medication** |
|---|---|
| Short-acting bronchodilators | Anticholinergic - ipratropium |
| | Beta-agonist - albuterol, fenoterol, terbutaline |
| Long-acting bronchodilators - twice daily | Anticholineric - glycopyrrolate, aclidinium |
| | Beta-agonist - salmeterol, formoterol |
| Long-acting bronchodilators - once daily | Anticholinergic - tiotropium, umeclidinium |
| | Beta-agonist - vilanterol, olodaterol |
| Inhaled steroids - twice daily | Fluticasone propionate, budesonide, mometasone, ciclesonide |
| Inhaled steroids - once daily | Fluticasone furoate |

In other embodiments, the handheld digital nebulizer device may be used for the systemic delivery of therapeutic agents including small molecules, therapeutic peptides, proteins, antibodies, and other bioengineered molecules via the pulmonary system. By way of non-limiting example, the handheld digital nebulizer device may be used to systemically deliver therapeutic agents for the treatment or prevention of indications inducing, e.g., diabetes mellitus, rheumatoid arthritis, plaque psoriasis, Crohn's disease, hormone replacement, neutropenia, nausea, influenza, etc.

By way of non-limiting example, therapeutic peptides, proteins, antibodies, and other bioengineered molecules include: growth factors, insulin, vaccines (Prevnor - Pneumonia, Gardasil - HPV), antibodies (Avastin, Humira, Remicade, Herceptin), Fc Fusion Proteins (Enbrel, Orencia), hormones (Elonva- long acting FSH, Growth Hormone), enzymes (Pulmozyme - rHu-DNAase- ), other proteins (Clotting factors, Interleukins, Albumin), gene therapy and RNAi, cell therapy (Provenge - Prostate cancer vaccine), antibody drug conjugates - Adcetris (Brentuximab vedotin for HL), cytokines, anti-infective agents, polynucleotides, oligonucleotides (e.g., gene vectors), or any combination thereof; or solid droplets or suspensions such as Flonase (fluticasone propionate) or Advair (fluticasone propionate and salmeterol xinafoate).

In other embodiments, the handheld digital nebulizer device of the disclosure may be used to deliver a solution of nicotine including the water-nicotine azeotrope for the delivery of highly controlled dosages for smoking cessation or a condition requiring medical or veterinary treatment. In addition, the fluid may contain THC, CBD, or other chemicals contained in marijuana for the treatment of seizures and other conditions.

In certain embodiments, the handheld digital nebulizer device of the disclosure may be used to deliver scheduled and controlled substances such as narcotics for the highly controlled dispense of pain medications where dosing is only enabled by doctor or pharmacy communication to the device, and where dosing may only be enabled in a specific location such as the patient's residence as verified by GPS location on the patient's smart phone. This mechanism of highly controlled dispensing of controlled medications can prevent the abuse or overdose of narcotics or other addictive drugs.

Certain benefits of the pulmonary route for delivery of drugs and other medications include a non-invasive, needle-free delivery system that is suitable for delivery of a wide range of substances from small molecules to very large proteins, reduced level of metabolizing enzymes compared to the GI tract and absorbed molecules do not undergo a first pass effect. (A. Tronde, et al., J Pharm Sci, 92 (2003) 1216-1233; A.L. Adjei, et al., Inhalation Delivery of Therapeutic Peptides and Proteins, M. Dekker, New York, 1997). Further, medications that are administered orally or intravenously are diluted through the body, while medications given directly into the lungs may provide concentrations at the target site (the lungs) that are about 100 times higher than the same intravenous dose. This is especially important for treatment of drug resistant bacteria, drug resistant tuberculosis, for example and to address drug resistant bacterial infections that are an increasing problem in the ICU.

Another benefit for giving medication directly into the lungs is that high, toxic levels of medications in the blood stream their associated side effects can be minimized. For example intravenous administration of tobramycin leads to very high serum levels that are toxic to the kidneys and therefore limits its use, while administration by inhalation significantly improves pulmonary function without severe side effects to kidney functions. (Ramsey et al., Intermittent administration of inhaled tobramycin in patients with cystic fibrosis. N Engl J Med 1999;340:23-30; MacLusky et al., Long-term effects of inhaled tobramycin in patients with cystic fibrosis colonized with Pseudomonas aeruginosa. Pediatr Pulmonol 1989;7:42-48; Geller et al., Pharmacokinetics and bioavailablility of aerosolized tobramycin in cystic fibrosis. Chest 2002;122:219-226.)

As discussed above, effective delivery of droplets deep into the lung airways require droplets that are less than about 5-6 microns in diameter, specifically droplets with mass mean aerodynamic diameters (MMAD) that are less than about 5 microns. The mass mean aerodynamic diameter is defined as the diameter at which 50% of the droplets by mass are larger and 50% are smaller. In certain aspects of the disclosure, in order to deposit in the alveolar airways, droplets in this size range must have momentum that is sufficiently high to permit ejection out of the device, but sufficiently low to overcome deposition onto the tongue (soft palate) or pharynx.

In other aspects of the disclosure, methods for generating an ejected stream of droplets for delivery to the pulmonary system of user using the handheld nebulizer devices of the disclosure are provided. In certain embodiments, the ejected stream of droplets is generated in a controllable and defined droplet size range. By way of example, the droplet size range includes at least about 50%, at least about 60%, at least about 70%, at least about 85%, at least about 90%, between about 50% and about 90%, between about 60% and about 90%, between about 70% and about 90%, etc., of the ejected droplets are in the respirable range of below about 5 µm.

In other embodiments, the ejected stream of droplets may have one or more diameters, such that droplets having multiple diameters are generated so as to target multiple regions in the airways (mouth, tongue, throat, upper airways, lower airways, deep lung, etc.) By way of example, droplet diameters may range from about 1 µm to about 200 µm, about 2 µm to about 100 µm, about 2 µm to about 60 µm, about 2 µm to about 40 µm, about 2 µm to about 20 µm, about 1 µm to about 5µm, about 1 µm to about 4.7 µm, about 1 µm to about 4 µm, about 10 µm to about 40 µm, about 10 µm to about 20 µm, about 5 µm to about 10 µm, and combinations thereof. In particular embodiments, at least a fraction of the droplets have diameters in the respirable range, while other droplets may have diameters in other sizes so as to target non-respirable locations (e.g., larger than 5 µm). Illustrative ejected droplet streams in this regard might have 50% - 70% of droplets in the respirable range (less than about 5 µm), and 30% -50% outside of the respirable range (about 5 µm - about 10 µm, about 5 µm - about 20 µm, etc.)

In another embodiment, methods for delivering safe, suitable, and repeatable dosages of a medicament to the pulmonary system using the handheld digital nebulizer device of the disclosure are provided. The methods deliver an ejected stream of droplets to the desired location within the pulmonary system of the subject, including the deep lungs and alveolar airways.

Suitable dosage and administration regimen may be determined based on the specific therapeutic agent or combination of agents to be administered to the subject in need thereof. As discussed herein, the present methods and devices allow for delivery of high concentrations of active agent directly to the pulmonary system of a subject. Suitable dosages and dosing regimens may be determined based, at least in part, on lung clearance properties of the therapeutic agent and desired therapeutic concentrations of the therapeutic agent at the site of interest (e.g., upper airways, lower airways, etc.). Many factors, including those described herein, can influence the desired dosage. Once the desired dosage is determined, and also if needed, desired frequency, such doses can be delivered. Frequency of dosing can vary by number of times, periodicity or both.

The term "therapeutically effective" amount refers to an amount of an active agent used to treat, ameliorate, prevent, or eliminate the identified condition (e.g., asthma, COPD, pneumonia, etc.), or to exhibit a detectable therapeutic or preventive effect. The effect can be detected by, for example, chemical markers, FEV1, lung capacity, or time to a measurable event, such as morbidity or mortality. The precise effective amount for a subject will depend upon the subject's body weight, size, and health; the nature and extent of the condition; and the therapeutic or combination of therapeutics selected for administration.

In certain aspects of the disclosure, a handheld nebulizer device for delivering an ejected stream of droplets to the pulmonary system of a subject is provided. The handheld nebulizer device generally includes a housing including an exhalation valve, a mouthpiece positioned at the airflow exit side of the housing, a reservoir disposed in or in fluid communication with the housing for receiving a volume of fluid, an ejector mechanism in fluid communication with the reservoir, and at least one differential pressure sensor positioned within the housing. The exhalation valve may be configured to allow for release of pressure during exhalation to thereby minimize back pressure through the device and ejector mechanism during use. The differential pressure sensor is configured to electronically breath activate the ejector mechanism upon sensing a pre-determined pressure change within the mouthpiece, and the ejector mechanism is configured to generate an ejected stream of droplets. The housing, its internal components, and various device components (e.g., the mouthpiece, air inlet flow element, etc.) are orientated in a substantially in-line or parallel configuration (e.g., along the airflow path) so as to form a small, hand-held nebulizer device.

In certain embodiments, the mouthpiece may be interfaced with (and optionally removable and/or replaceable), integrated into, or part of the housing. In other embodiments, the mouthpiece may be interfaced with (and optionally removable and/or replaceable), integrated into, or part of the drug delivery ampoule.

The ejector mechanism may include a piezoelectric actuator which is directly or indirectly coupled to an aperture plate having a plurality of openings formed through its thickness. The piezoelectric actuator is operable to directly or indirectly oscillate the aperture plate at a frequency to thereby generate an ejected stream of droplets.

In certain embodiments, the housing and ejector mechanism are oriented such that the exit side of aperture plate is perpendicular to the direction of airflow and the stream of droplets is ejected in parallel to the direction of airflow. In other embodiments, the housing and ejector mechanism are oriented such that the exit side of aperture plate is parallel to the direction of airflow and the stream of droplets is ejected substantially perpendicularly to the direction of airflow such that the ejected stream of droplets is directed through the housing at an approximate 90 degree change of trajectory prior to expulsion from the housing.

In certain embodiments, the handheld nebulizer device is comprised of a separate drug delivery ampoule with an ejector mechanism (e.g., combination reservoir/ejector mechanism module) embedded within a surface of a drug reservoir, and a handheld base unit (e.g., housing) including a differential pressure sensor, a microprocessor and three AAA batteries. In certain embodiments, the handheld base unit also includes a mouthpiece, optionally removable, an optional mouthpiece cover, and an optional ejector plate seal. The microprocessor controls dose delivery, dose counting and software designed monitoring parameters that can be transmitted through wireless communication, e.g., Wi-Fi, cellular, blue-tooth technology, etc. The ejector mechanism optimizes droplet delivery to the lungs by creating an ejected droplet stream in a predefined range with a high degree of accuracy and repeatability. Initial droplet studies show at least 65% to 70% of droplets ejected from the device are in the respirable range (e.g., 1-5 µm).

In certain embodiments, the handheld nebulizer device may include a combination reservoir/ejector mechanism module (e.g., drug delivery ampoule) that may be replaceable or disposable either on a periodic basis, e.g., a daily, weekly, monthly, as-needed, etc. basis, as may be suitable for a prescription or over-the-counter medication. The reservoir may be prefilled and stored in a pharmacy for dispensing to patients or filled at the pharmacy or elsewhere by using a suitable injection means such as a hollow injection syringe driven manually or driven by a micro-pump. The syringe may fill the reservoir by pumping fluid into or out of a rigid container or other collapsible or non-collapsible reservoir. In certain aspects, such disposable/replaceable, combination reservoir/ejector mechanism module may minimize and prevent buildup of surface deposits or surface microbial contamination on the aperture plate, owing to its short in-use time.

In another embodiment of the disclosure, the handheld digital nebulizer device may include two or more, three or more, four or more reservoirs, e.g., a dual or multiple reservoir configuration. In certain embodiments, the dual or multiple reservoirs may be a combination dual or multiple reservoir/ejector module configuration, which may be removable and/or disposable. The dual or multiple reservoirs can deliver multiple medications, flavors, or a combination thereof for polypharmacy.

In other embodiments, the handheld nebulizer device of the disclosure may include a small volume drug ampoule, e.g., configured as a single use ampoule (e.g., disposable on a daily or on-use basis). Such embodiments are particularly useful with therapeutic agents that are sensitive to storage conditions, e.g., sensitive to degradation, aggregation, conformational changes, contamination, etc. In this regard, the small volume drug ampoule allows for sterile storage of a therapeutic agent under appropriate conditions until the time of use, e.g., under a temperature controlled environment, as a powder-for-reconstitution, etc. By way of non-limiting example, the small volume drug ampoule of the disclosure is particular suitable for use with therapeutic peptides, proteins, antibodies, and other bioengineered molecules or biologics. However, the disclosure is not so limited, and the small volume drug ampoule may be used with any therapeutic agent known in the art.

Without intending to be limited by theory, in certain aspects, the small volume drug ampoule of the disclosure may offer advantages over larger volume/multi-use ampoules in that, e.g., the limited duration of use minimizes evaporation of fluid in the reservoir, minimizes the possibility of contamination of fluid in the reservoir and/or the ejector surface, minimizes the duration of time of the ampoule is held at non-controlled storage conditions, etc.

In certain embodiments, the small volume drug ampoule includes a drug reservoir for receiving a small volume of fluid, e.g., a volume equivalent to 10 or fewer dosages, a volume equivalent to 5 or fewer dosages, a volume equivalent to 4 or fewer dosages, a volume equivalent to 3 or fewer dosages, a volume equivalent to 2 or fewer dosages, a single dose volume. The small volume drug ampoule is configured to facilitate the ejection of small, e.g., single use, volumes of a therapeutic agent.

In certain embodiments, the small volume drug ampoule may include a reservoir which comprises an internal flexible membrane separating two internal volumes, a first background pressure fluid volume and a second drug volume. In certain aspects, the membrane separates the two volumes such that the background pressure fluid volume creates an area of fluid behind/above the drug volume without allowing mixing or diluting of the therapeutic agent by the background pressure fluid. The small volume drug ampoule may further comprise an air exchange vent or air space in the region of the background pressure fluid volume, configured to prevent or relieve the creation of negative pressure during ejection of the drug fluid during use. The air exchange vent may include a superhydrophobic filter, optionally in combination with a spiral vapor barrier, which provides for free exchange of air into and out of the reservoir.

In certain aspects of the disclosure, the ejector mechanism, reservoir, and housing/mouthpiece function to generate a plume with droplet diameters less than about 5 um. As discussed above, in certain embodiments, the reservoir and ejector mechanism modules are powered by electronics in the device housing and a reservoir which may carry sufficient drug for a single dose, just a few doses, or several hundred doses of medicament.

The present disclosure also provides a handheld nebulizer device that is altitude insensitive. In certain implementations, the handheld nebulizer device is configured so as to be insensitive to pressure differentials that may occur when the user travels from sea level to sub-sea levels and at high altitudes, e.g., while traveling in an airplane where pressure differentials may be as great as 4 psi. As will be discussed in further detail herein, in certain implementations of the disclosure, the handheld nebulizer device may include a superhydrophobic filter, optionally in combination with a spiral vapor barrier, which provides for free exchange of air into and out of the reservoir, while blocking moisture or fluids from passing into the reservoir, thereby reducing or preventing fluid leakage or deposition on aperture plate surfaces.

In certain aspects, the devices of the disclosure eliminate the need for patient / device coordination by using a differential pressure sensor to initiate the piezoelectric ejector in response to the onset of inhalation. The device does not require manual triggering of medication delivery. Unlike propellant driven MDIs, the droplets from the devices of the disclosure are generated having little to no intrinsic velocity from the aerosol formation process and are inspired into the lungs solely by the user's incoming breath passing through the mouthpiece. The droplets will ride on entrained air providing improved deposition in the lung.

In certain embodiments, as described in further detail herein, when the drug ampoule is mated to the handheld base unit, electrical contact is made between the base containing the batteries and the ejector mechanism embedded in the drug reservoir. In certain embodiments, visual indications, e.g., a horizontal series of three user visible LED lights, and audio indications via a small speaker within the handheld base unit may provide user notifications. By way of example, the device may be, e.g., 2.0 -3.5 cm high, 5-7 cm wide, 10.5-12 cm long and may weight approximately 95 grams with an empty drug ampoule and with batteries inserted.

As described herein, in certain embodiments, the handheld nebulizer device may be turned on and activated for use by inserting the drug ampoule into the base unit, opening the mouthpiece cover, and/or switching an on/off switch/slide bar. In certain embodiments, visual and/or audio indicators may be used to indicate the status of the device in this regard, e.g., on, off, stand-by, preparing, etc. By way of example, one or more LED lights may turn green and/or flash green to indicate the device is ready for use. In other embodiments, visual and/or audio indicators may be used to indicate the status of the drug ampoule, including the number of doses taken, the number of doses remaining, instructions for use, etc. For example, and LED visual screen may indicate a dose counter numerical display with the number of remaining doses in the reservoir.

As described in further detail herein, during use as a user inhales through the mouthpiece of the housing of a handheld nebulizer device of the disclosure, a differential pressure sensor within the housing detects inspiratory flow, e.g., by measuring the pressure drop across a Venturi plate at the back of the mouthpiece. When a threshold pressure decline (e.g., 8 slm) is attained, the microprocessor activates the ejector mechanism, which in turn generates an ejected stream of droplets into the airflow of the device that the user inhales through the mouthpiece. In certain embodiments, audio and/or visual indicates may be used to indicate that dosing has been initiated, e.g., one or more LEDs may illuminate green. The user then continues to inhale and exhale through the device for a desired dosing during (the exhalation valve configured such that pressure does not build up through the device during exhalation, as described herein). The microprocessor then deactivates the ejector at a designated time after initiation so as to achieve a desired administration dosage, e.g., 1 second to 10 minutes. In certain embodiments, the microprocessor deactivates the ejector during the user's exhalation phase (or at a designated time that corresponds to an expected exhalation cycle), e.g., 1-1.45 seconds, and then reinitiates activation during the next inspiration cycle, e.g., upon sensing of a pressure drop within the housing of the handheld nebulizer.

In certain embodiments, the handheld nebulizer may eject droplets for only a one breath (e.g., 1-1.45 seconds, etc.), the handheld nebulizer may continue to eject droplets for an extended treatment time, (several seconds to several minutes, e.g., 2 seconds, 5 seconds, 10 seconds, 30 seconds, 1 minute, 2 minutes, 5 minutes, 10 minutes, 15 minutes, 20 minutes, etc.), or the handheld nebulizer may cycle ejections such that the ejector is activated during inhalation cycles of the user (and the ejector is deactivated during exhalation cycles of the user). The duration of administration of droplets will depend on the desired dosage, as recognized by those of skill in the art.

In certain embodiments, as described in further detail herein, the device may provide visual and/or audio indicators to facilitate proper dosing, e.g., the device may emit a positive chime sound after the initiation of dosing, indicating to the user to begin holding their breath for a designated period of time, e.g., 10 seconds. During the breath hold period, e.g., the three green LEDs may blink. Additionally, there may be voice commands instructing the patient on proper times to exhale, inhale and hold their breath, with an audio indicator of a breath hold countdown.

As described above, in certain embodiments, it may be desired for the ejector to be deactivated during exhalation cycles of the user so as to, e.g., minimize drug waste and/or ejection when the therapeutic agent is not being inhaled by the user. In this regard, microprocessor of the handheld nebulizer device may include control logic and feedback sensors such that the ejector mechanism is deactivated whenever the device and pressure sensors determine that the user is not actively inhaling through the device. Alternatively, the microprocessor may be set with predetermined activation times following a sensing of a pressure drop through the device. Together with the exhalation valve located on the housing/mouthpiece, the user is able to freely inhale and exhale through the device, and the handheld nebulizer device will efficiently deliver therapeutic agent in an automated manner while minimizing waste.

Following dosing, the handheld nebulizer device may turned off and deactivated in any suitable manner, e.g., by closing the mouthpiece cover, switching an on/off switch/slide bar, timing out from non-use, removing the drug ampoule, etc. If desired, audio and/or visual indicators may prompt a user to deactivate the device, e.g., by flashing one or more red LED lights, providing voice commands to close the mouthpiece cover, etc.

In certain embodiments, the handheld nebulizer device may include an ejector mechanism closure system that seals the aperture plate when not in use to protect the integrity of the aperture plate and to minimize and prevent contamination and evaporation of the fluid within the reservoir. For example, in some embodiments, the device may include a mouthpiece cover that comprises a rubber plug that is sized and shaped to seal the exit side surface of the aperture plate when the cover is closed. In other embodiments, the mouthpiece cover may trigger a slide to seal the exit side surface of the aperture plate when the cover is closed. Other embodiments and configurations are also envisioned, e.g., manual slides, covers, and plugs, etc. In certain aspects, the microprocessor may be configured to detect when the ejector mechanism closure, aperture plate seal, etc. is in place, and may thereafter deactivate the device.

Several features of the device allow precise dosing of specific droplet sizes. Droplet size is set by the diameter of the holes in the mesh which are formed with high accuracy. By way of example, the holes in the aperture plate may range in size from 1 µm to 6 µm, from 2 µm to 5 µm, from 3 µm to 5 µm, from 3 µm to 4 µm, etc. Ejection rate, in droplets per second, is generally fixed by the frequency of the aperture plate vibration, e.g., 108-kHz, which is actuated by the microprocessor. In certain embodiments, there is less than a 50-millisecond lag between the detection of the start of inhalation and full droplet generation.

Other aspects of the device of the disclosure that allow for precise dosing of specific droplet sizes include the production of droplets within the respirable range early in the inhalation cycle, thereby minimizing the amount of drug product being deposited in the mouth or upper airways at the end of an inhalation. In addition, the design of the drug ampoule allows the aperture plate surface to be wetted and ready for ejection without user intervention, thus obviating the need for shaking and priming. Further, the design of the drug ampoule vent configuration together with the ejector mechanism closure system limits fluid evaporation from the reservoir to less than 150 µL to 350 µL per month.

The device may be constructed with materials currently used in FDA cleared devices. Standard manufacturing methods may be employed to minimize extractables.

Any suitable material may be used to form the housing of the handheld nebulizer device. In particular embodiment, the material should be selected such that it does not interact with the components of the device or the fluid to be ejected (e.g., drug or medicament components). For example, polymeric materials suitable for use in pharmaceutical applications may be used including, e.g., gamma radiation compatible polymer materials such as polystyrene, polysulfone, polyurethane, phenolics, polycarbonate, polyimides, aromatic polyesters (PET, PETG), etc.

The drug ampoule may be constructed of any suitable materials for the intended pharmaceutical use. In particular, the drug contacting portions may be made from material compatible with the desired active agent(s), e.g., albuterol sulfate and ipratropium bromide. By way of example, in certain embodiments, the drug only contacts the inner side of the drug reservoir and the inner face of the aperture plate and piezoelectric element. Wires connecting the piezoelectric ejector mechanism to the batteries contained in the base unit may be embedded in the drug ampoule shell to avoid contact with the drug. The piezoelectric ejector may be attached to the drug reservoir by a flexible bushing. To the extent the bushing may contact the drug fluid, it may be, e.g., any suitable material known in the art for such purposes such as those used in piezoelectric nebulizers.

In certain embodiments, the device mouthpiece may be removable, replaceable and may be cleaned. Similarly, the device housing and drug ampoule can be cleaned by wiping with a moist cloth. In certain embodiments, the mouthpiece may be interfaced with (and optionally removable and/or replaceable), integrated into, or part of the housing. In other embodiments, the mouthpiece may be interfaced with (and optionally removable and/or replaceable), integrated into, or part of the drug delivery ampoule.

Again, any suitable material may be used to form the mouthpiece of the handheld nebulizer device. In particular embodiment, the material should be selected such that it does not negatively interact with the components of the device or the fluid to be ejected (e.g., drug or medicament components). For example, polymeric materials suitable for use in pharmaceutical applications may be used including, e.g., gamma radiation compatible polymer materials such as polystyrene, polysulfone, polyurethane, phenolics, polycarbonate, polyimides, aromatic polyesters (PET, PETG), etc. In certain embodiments, the mouthpiece may be removable, replaceable and sterilizable. This feature improves sanitation for drug delivery by providing a mechanism to minimize buildup of aerosolized medication within the mouthpiece and by providing for ease of replacement, disinfection and washing. In one embodiment, the mouthpiece tube may be formed from sterilizable and transparent polymer compositions such as polycarbonate, polyethylene or polypropylene, as discussed herein.

In certain aspects of the disclosure, an electrostatic coating may be applied to the one or more portions of the housing, e.g., inner surfaces of the housing along the airflow pathway such as the mouthpiece, to aid in reducing deposition of ejected droplets during use due to electrostatic charge build-up. Alternatively, one or more portions of the housing may be formed from a charge-dissipative polymer. For instance, conductive fillers are commercially available and may be compounded into the more common polymers used in medical applications, for example, PEEK, polycarbonate, polyolefins (polypropylene or polyethylene), or styrenes such as polystyrene or acrylic-butadiene-styrene (ABS) copolymers. Alternatively, in certain embodiments, one or more portions of the housing, e.g., inner surfaces of the housing along the airflow pathway such as the mouthpiece, may be coated with anti-microbial coatings, or may be coated with hydrophobic coatings to aid in reducing deposition of ejected droplets during use. Any suitable coatings known for such purposes may be used, e.g., polytetrafluoroethylene (Teflon).

Any suitable differential pressure sensor with adequate sensitivity to measure pressure changes obtained during standard inhalation cycles may be used, e.g., ± 5 SLM, 10 SLM, 20 SLM, etc. For instance, pressure sensors from Sensirion, Inc., SDP31 or SDP32 (US 7,490,511 B2) are particularly well suited for these applications.

In certain aspects, the microprocessor in the device may be programmed to ensure exact timing and actuation of the ejector mechanism in accordance with desired parameters, e.g., based duration of piezoelectric activation to achieve desired dosages, etc. In certain embodiments, the device includes or interfaces with a memory (on the device, smartphone, App, computer, etc.) to record the date-time of each ejection event, as well as the user's inhalation flow rate during the dose inhalation to facilitate user monitoring, as well as drug ampoule usage monitoring. For instance, the microprocessor and memory can monitor doses administered and doses remaining in a particular drug ampoule. In certain embodiments, the drug ampoule may comprise components that include identifiable information, and the base unit may comprise components that may "read" the identifiable information to sense when a drug ampoule has been inserted into the base unit, e.g., based on a unique electrical resistance of each individual ampoule, an RFID chip, or other readable microchip (e.g., cryptoauthentication microchip). Dose counting and lockouts may also be preprogramed into the microprocessor.

In certain embodiments of the present disclosure, the signal generated by the pressure sensors provides a trigger for activation and actuation of the ejector mechanism to thereby generate droplets and delivery droplets at or during a peak period of a patient's inhalation (inspiratory) cycle and assures optimum deposition of the plume of droplets and delivery of the medication into the pulmonary airways of the user.

In accordance with certain aspects of the disclosure, the handheld nebulizer device provides a reliable monitoring system that can date and time stamp actual deliver of medication, e.g., to benefit patients through self-monitoring or through involvement of care givers and family members.

As described in further detail herein, the handheld nebulizer device of the disclosure may detect inspiratory airflow and record/store inspiratory airflow in a memory (on the device, smartphone, App, computer, etc.). A preset threshold (e.g., 8-10 slm) triggers delivery of medication over a defined period of time, e.g., 1 second - 10 minutes, via inhalation/exhalation cycling as described herein, etc., depending on the needs of the nebulizer. Inspiratory flow is sampled frequently until flow stops. The number of times that delivery is triggered is incorporated and displayed in the dose counter LED on the device. Blue tooth capabilities permit the wireless transmission of the data.

Wireless communication in the device will communicate date, time and number of actuations per session to the user's smartphone. Software programing can provide charts, graphics, medication reminders and warnings to patients and whoever is granted permission to the data. The software application will be able to incorporate multiple medications that use the device of the disclosure.

The device of the disclosure can also provide directed instruction to users, including audio and visual indicators to facilitate proper use of the device and proper dosing. For instance, certain patients that may need drug to be delivered to an inflamed and narrowed lower respiratory region are typically asked to inhale drug particles slowly and steadily followed by about ten seconds of holding their breath to allow sedimentation to occur. In a medical office these patients can be coached and encouraged to hold their breath after inhalation. However, outside of a medical care setting, improper use of an inhaler device often results.

The device of the present disclosure is configured to dispense droplets during the correct part of the inhalation cycle, and can including instruction and/or coaching features to assist patients with proper device use, e.g., by instructing the holding of breath for the correct amount of time after inhalation. The device of the disclosure allows this dual functionality because it may both monitor air flow during the inhalation, and has internal sensors/controls which may detect the end of inhalation (based upon measured flow rate) and can cue the patient to hold their breath for a fixed duration after the inhalation ceases.

In one exemplary embodiment, a patient may be coached to hold their breath with an LED that is turned on at the end of inhalation and turned off after a defined period of time (i.e., desired time period of breath hold), e.g., 10 seconds. Alternatively, the LED may blink after inhalation, and continue blinking until the breath holding period has ended. In this case, the processing in the device detects the end of inhalation, turns on the LED (or causes blinking of the LED, etc.), waits the defined period of time, and then turns off the LED. Similarly, the device can emit audio indications, e.g., one or more bursts of sound (e.g., a 50 millisecond pulse of 1000 Hz), verbal instructions to hold breath, verbal countdown, music, tune, melody, etc., at the end of inhalation to cue a patient to hold their breath for the during of the sound signals. If desired, the device may also vibrate during or upon conclusion of the breath holding period.

In certain embodiments, the device provides a combination of audio and visual methods (or sound, light and vibration) described above to communicate to the user when the breath holding period has begun and when it has ended. Or during the breath holding to show progress (e.g., a visual or audio countdown).

In other aspects, the device of the disclosure may provide coaching to inhale longer, more deeply, etc. The average peak inspiratory flow during inhalation (or dosing) can be utilized to provide coaching. For example, a patient may hear a breath deeper command until they reach 90% of their average peak inspiratory flow as measured during inspiration (dosing) as stored on the device, phone or in the cloud.

In addition, an image capture device, including cameras, scanners, or other sensors without limitation, e.g. charge coupled device (CCD), may be provided to detect and measure the ejected aerosol plume. These detectors, LED, delta P transducer, CCD device, all provide controlling signals to a microprocessor or controller in the device used for monitoring, sensing, measuring and controlling the ejection of a plume of droplets and reporting patient compliance, treatment times, dosage, and patient usage history, etc., via Bluetooth^{®}, for example.

Reference will now be made to the figures, with like components illustrates with like references numbers.

**FIGS. 1A** and **1B** illustrate an exemplary handheld nebulizer device of the disclosure, with **FIG. 1A** showing the handheld nebulizer device 100 having a mouthpiece cover 102 in the closed position, and **FIG. 1B** having a mouthpiece cover 102 in the open position. As shown, the handheld nebulizer device is configured in an in-line orientation in that the housing, its internal components, and various device components (e.g., the mouthpiece, air inlet flow element, etc.) are orientated in a substantially in-line or parallel configuration (e.g., along the airflow path) so as to form a small, hand-held device.

In the embodiment shown in **FIGS. 1A** and **1B****,** the handheld nebulizer device 100 includes a base unit 104 and a drug delivery ampoule 106. As illustrated in this embodiment, and discussed in further detail herein, the drug delivery ampoule 106 slides into the front of the base unit 104 via slides 112. In certain embodiments, mouthpiece cover 102 may include a push element 102a that facilitates insertion of drug delivery ampoule 106. Also illustrated are one or more airflow entrances or openings 110. By way of example, there may be airflow entrances on the opposite side of the device, multiple airflow entrances on the same side of the device, or a combination thereof (not shown). The handheld nebulizer device 100 also includes mouthpiece 108 at the airflow exit side of the device.

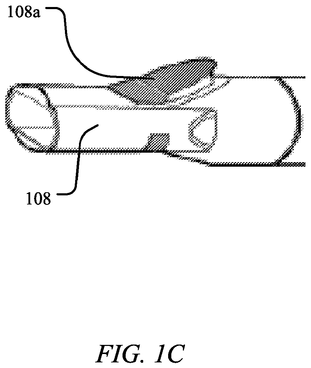

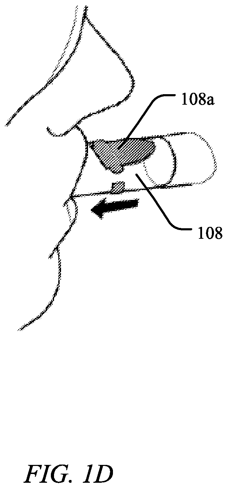

Although not shown in the embodiment illustrated in **FIGS. 1A** and **1B****,** the mouthpiece 108 at the airflow exit side of the device **(****FIG. 1D****)** includes an exhalation valve 108a, as illustrated in **FIGS. 1C** and **1D****.** In this regard, the mouthpiece shown in **FIGS. 1C** and **1D** including exhalation valve 108a may be included as the mouthpiece of any of the handheld nebulizer devices illustrated herein, including mouthpiece 108 of device 100 of **FIGS. 1A** and **1B****,** or any of the other embodiments disclosed herein. The exhalation valve may be configured to allow for release of pressure during exhalation to thereby minimize back pressure through the device and ejector mechanism. During use, on exhalation, the exhalation valve will open to release exhalation pressure and minimize any back pressure caused by the exhalation breath through the device and ejector mechanism.

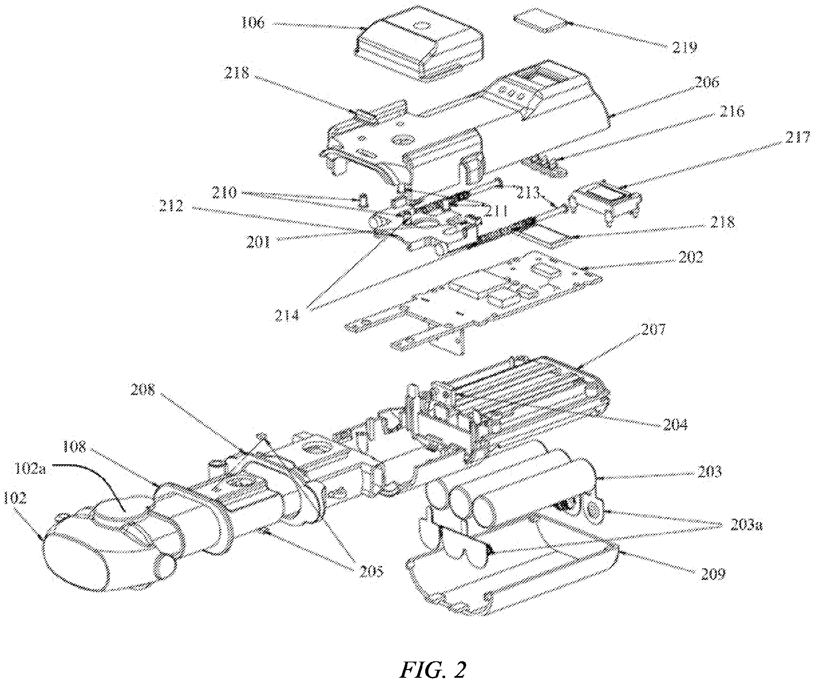

With reference to **FIG. 2****,** an exploded view of the exemplary handheld nebulizer device of **FIGS. 1A** and **1B** is shown, including internal components of the housing including a power/activation button 201; an electronics circuit board 202; a drug delivery ampoule 106 that comprises an ejector mechanism and reservoir (not shown); and a power source 203 (e.g., three AAA batteries, which may optionally be rechargeable) along with associated contacts 203a. In certain embodiments, the reservoir may be single-unit dose or multi-unit dose that may be replaceable, disposable or reusable. Also shown, one or more pressure sensors 204 and optional spray sensors 205. In certain embodiments, the device may also include various electrical contacts 210 and 211 to facilitate activation of the device upon insertion of drug delivery ampoule 106 into the base unit. Likewise, in certain embodiments, the device may include slides 212, posts 213, springs 214, and ampoule lock 215 to facilitate insertion of drug delivery ampoule 106 into the base unit.

The components may be packaged in a housing, and generally oriented in an in-line configuration. Again, the housing may include an exhalation valve (not shown), as illustrated in **FIGS. 1C** and **1D****.** The housing may be disposable or reusable, single-dose or multi-dose. Although various configurations to form the housing are within the scope of the disclosure, as illustrated in **FIG. 2****,** the housing may comprise a top cover 206, a bottom cover 207, and an inner housing 208. The housing may also include a power source housing or cover 209.

In certain embodiments, the device may include audio and/or visual indications, e.g., to provide instructions and communications to a user. In such embodiments, the device may include a speaker or audio chip (not shown), one or more LED lights 216, and LCD display 217 (interfaced with an LCD control board 218 and lens cover 219). The housing may be handheld and may be adapted for communication with other devices via a Bluetooth^{®} communication module or similar wireless communication module, e.g., for communication with a subject's smart phone, tablet or smart device (not shown).

In certain embodiments, an air inlet flow element (not shown, see, e.g., FIGS. 5A-5C and **FIGS. 11A****-18D)** may be positioned in the airflow at the airflow entrance of the housing and configured to facilitate non-turbulent (i.e., laminar and/or transitional) airflow across the exit side of aperture plate and to provide sufficient airflow to ensure that the ejected stream of droplets flows through the handheld nebulizer device during use. In some embodiments, the air inlet flow element may be positioned within the mouthpiece. Aspects of the present embodiment further allows customizing the internal pressure resistance of the particle delivery device by allowing the placement of laminar flow elements having openings of different sizes and varying configurations to selectively increase or decrease internal pressure resistance, as will be explained in further detail herein.

By way of non-limiting example, an exemplary method of insertion of an ampoule through to use and powering off of the device may be performed as follows:
1. When a new ampoule is initially inserted and pushed onto the device slide guide the device door is open and the ampoule slides and clicks into ampoule position 1. At this setting, an aperture plate seal or cover on the ampoule is open and electrical contacts on the device and ampoule make contact. The system is powered ON and ready for breath actuation. When the device door is opened, an audible beep may be emitted and LED indicator(s) may turn green or flash to notify the user that the system is ON and ready for dosing by inhaling through the mouthpiece.
2. As a patient inhales, a pre-set pressure value is reached and detected by the pressure sensor located within the housing (e.g., delta P sensor) and a second audible indicator or LED indicator may now indicate that a dose is triggered. After the dose is triggered, the device is activated and continues to eject as the subject inhales and exhales through the device (with exhalation pressure relieved through the exhalation valve), another audible and/or LED indicator may trigger until a spray cycle time of, e.g., 1 second to 10 minutes, via inhalation/exhalation cycling as described herein (or other designated dosing time) ends. Further, if desired, when a desired dose is delivered, the dose counter displayed on the LCD will indicate that a dose was delivered by a decrease in number of doses displayed on the LCD.
3. If no additional doses are required and an inactivity time of, e.g., 15 seconds to 5 minutes elapse, an audible and/or LED indicator may trigger to alert the user that the device is about to power-off, after which time the device may enter into a low power, sleep mode.
4. If no additional doses are required, the device door is closed to push the ampoule to the non-use position, the aperture plate seal or cover is closed and the device is in placed sleep mode. Further, as the slide mechanism releases pressure from the ON/OFF switch, and the system is now OFF.
5. When a patient is ready to apply additional doses, the device door is opened and the ampoule slides towards the mouthpiece as it is pushed by a spring-loaded mechanism from the non-use position to the use position, to thereby open the aperture plate seal or cover.

More particularly, a specific exemplary embodiment of a mode of operation of insertion of a drug ampoule and operation of a device is illustrated in **FIGS. 3A-1 to FIG. 3C-3**. Referring to **FIG. 3A-1** and **3A-2**, when a drug ampoule (1), is initially inserted and pushed onto the device slide guide (1a), the device door (2) is open, the ampoule slides and clicks into ampoule position 1. An oval button (ampoule lock) (1b) clicks down and snaps back to lock the ampoule in place. At this setting, the seal on the aperture plate is open, the four electrical contacts on the device and ampoule make contact, and the system is powered ON, ready for breath actuation. The front two contacts (3) complete the circuit to actuate the piezoelectric element, while the rear two contacts (4) are used to provide specific information on the ampoule, such as ampoule ID, drug type, dosage, etc.

Referring to **FIG. 3B-1** and **3B-2**, ampoule position 1(A) is shown, in which the oval button (1b) locks the ampoule into place and the four electrical contacts, front (3) and rear (4) connect to complete the electric circuit. When the ampoule is in position 1, the electronic component that activates the ON/OFF button (1c) is pushed by the spring-loaded, slide mechanism (5). **FIG. 3B-1** provides a bottom view of the spring-loaded slide mechanism (5) and the ON/OFF button (1c), in the ON mode. **FIG. 3B-2** provides an exploded view (5a) of side brackets on the spring-loaded slide (5) and their position (5a- dash arrows) through slots (5b) on the device which make contact on the ampule (5c) to push the ampule forward when the device door is opened and activate the ON/OFF switch (1c) as it makes contact with the ON/OFF button (1d). The device ON/OFF button (1c) is activated by the slide (5) when the mouthpiece cover (2) is closed and pushes the ampule back to position 2, where the aperture plate seal is in the closed position and power is turned OFF to the device as pressure on the ON/OFF switch is released.

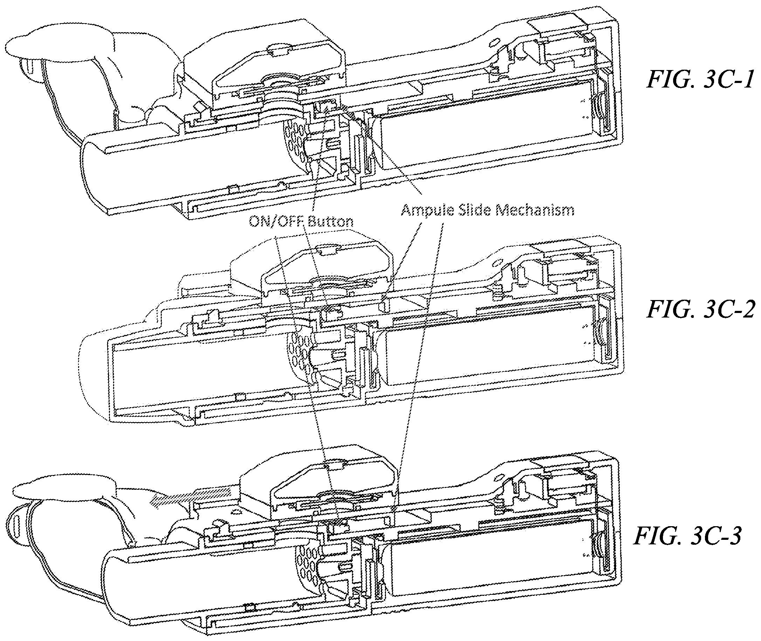

Referring to **FIG. 3C-1**, **3C-2**, and **3C-3,** cross-sections of the device with the ampoule inserted are illustrated to better illustrate the ampoule slide mechanism and positioning of the ON/OFF switch. **FIG. 3C-1** shows ampoule position 1, with the mouthpiece cover in the open position and the ON/OFF switch in the ON position. **FIG. 3C-2** shows ampoule position 2, with the mouthpiece cover in the closed position and the ON/OFF switch in the OFF position. **FIG. 3C-3** shows ampoule position 2, with the mouthpiece cover in the open position and the ON/OFF switch in the OFF position.

However, it is noted that the devices and methods of the disclosure are not so limited, and various modifications and expansions of the method of operation is envisioned as within the scope of the disclosure.

In another embodiment, **FIGS. 4A** and **4B** illustrate an alternative handheld nebulizer device of the disclosure, with **FIG. 4A** showing the handheld nebulizer device 400 with a base unit 404 having a mouthpiece cover 402 in the closed position, and **FIG. 4B** with a base unit 404 having a mouthpiece cover 402 in the open position. As shown, the handheld nebulizer device is configured in an in-line orientation in that the housing, its internal components, and various device components (e.g., the mouthpiece, air inlet flow element, etc.) are orientated in a substantially in-line or parallel configuration (e.g., along the airflow path) so as to form a small, hand-held device.

In the embodiment shown in **FIGS. 4A** and **4B****,** the handheld nebulizer device 400 includes a base unit 404 and a drug delivery ampoule 406. As illustrated in this embodiment, and discussed in further detail herein, the drug delivery ampoule 406 slides into the front of the base unit 404. In certain embodiments, mouthpiece cover 402 may include aperture plate plug 412. Also illustrated are one or more airflow entrances or openings 410 in mouthpiece 408. By way of example, there may be airflow entrances on the opposite side of the device, multiple airflow entrances on the same side of the device, or a combination thereof (not shown). The handheld nebulizer device 400 also includes mouthpiece 408 at the airflow exit side of the device.

Again, although not shown in the embodiment illustrated in **FIGS. 4A** and **4B****,** the mouthpiece 108 shown in **FIGS. 1C** and **1D** including exhalation valve 108a may be included as the mouthpiece of any of the handheld nebulizer devices illustrated herein, including mouthpiece 408 of device 400 of **FIGS. 4A** and **4B****,** or any of the other embodiments disclosed herein.

With reference to **FIG. 5****,** an exploded view of the exemplary handheld nebulizer device of **FIGS. 4A** and **4B** is shown, including internal components of the housing including an electronics circuit board 502; a drug delivery ampoule 406 that comprises top cover 430 having optional vents 431 and vapor barriers 432, an ejector mechanism 434, a drug reservoir 435, electrical contacts 436, and one or more sensor ports 437; and a power source 503 (e.g., three AAA batteries, which may optionally be rechargeable). In certain embodiments, the device may also include various electrical contacts 442 and sensor ports 444 to facilitate activation of the device upon insertion of drug delivery ampoule 406 into the base unit 404. Likewise, in certain embodiments, the device may include resistors or chips 504 to facilitate insertion and detection of drug delivery ampoule 406 into the base unit 404.

In certain embodiments, the reservoir may be single-unit dose or multi-unit dose that may be replaceable, disposable or reusable. As illustrated in **FIG. 5****,** in certain embodiments, the drug delivery ampoule may also comprise or be interfaced with a mouthpiece 408 and a mouthpiece cover 402. As shown, ejector mechanism 434 may be positioned in line with mouthpiece 408 and drug reservoir 435 such that the exit side of the aperture plate is perpendicular to the direction of airflow and the stream of droplets is ejected in parallel to the direction of airflow. The mouthpiece cover 402 may further include an aperture plate plug 412.

The components may be packaged in a housing, and generally oriented in an in-line configuration. Again, the housing may include an exhalation valve. The housing may be disposable or reusable, single-dose or multi-dose. Although various configurations to form the housing are within the scope of the disclosure, as illustrated in **FIG. 5****,** the housing may comprise a top cover 506, a bottom cover 507, and an inner housing 508. The device may also include one or more ampoule release buttons 550, e.g., positioned on the side of the housing to facilitate release of the drug delivery ampoule 406 once inserted into the base unit 404.

In certain embodiments, the device may include audio and/or visual indications, e.g., to provide instructions and communications to a user. In such embodiments, the device may include a speaker or audio chip 520, one or more LED lights 516, and LCD display 517 (interfaced with an LCD control board 518 and lens cover 519). The housing may be handheld and may be adapted for communication with other devices via a Bluetooth^{®} communication module or similar wireless communication module, e.g., for communication with a subject's smart phone, tablet or smart device (not shown).

With reference to **FIG. 6****,** a cross-section of an in-line device of **FIGS. 4A** and **4B** is shown to illustrate an exemplary configuration of the interior of the drug reservoir 435 and its relation to ejector mechanism 434. As shown, drug reservoir 435 may be sized and shaped such that the volume of fluid held within the reservoir is funneled and directed to the ejection surface of the aperture plate during use. More particularly, as shown, the bottom surface of the drug reservoir may be sloped towards the ejector mechanism so as to facilitate flow of the fluid within the drug reservoir during use. Without intending to be limited by theory, such configurations may be particularly suited for device orientations wherein the ejector mechanism is oriented perpendicularly to the direction of airflow. However, it is noted that the disclosure is not so limited, and various shapes, sizes and configurations of ampoule are envisioned as within the scope of the disclosure.

**FIG. 7** illustrates the base unit 404 of the embodiment of **FIGS. 4A** and **4B** without the drug delivery ampoule inserted. Without the drug delivery ampoule inserted, tracks 440 for directing the ampoule into place, electrical contacts 442, and sensor port 444 are shown. Also shown is release button 450.

**FIGS. 8A** and **8B** illustrate a drug delivery ampoule 406 with mouthpiece cover 402 attached and in a closed position in front view **(****FIG. 8A****)** and back view **(****FIG. 8B). FIG. 8B** illustrates electrical contacts 436 and sensor port 437 of the ampoule, as well as protruding slides 452 to facilitate placement of the ampoule into tracks 440 during insertion. By way of example, when drug delivery ampoule 406 is inserted into base unit 404, protruding slides 452 mate with tracks 440, sensor port 437 mates with sensor port 444, and electrical contacts 436 mates with electrical contacts 442. The drug delivery ampoule is pushed into the base unit and locked into place with the protruding slides and tracks engaging one another. During use, a pressure sensor located on the control board senses pressure changes within the device via the pressure sensing ports (e.g., within the mouthpiece). To facilitate detection of pressure changes, the base unit includes a second pressure sensing port and outside channel (not shown) to facilitate sensing of reference or ambient pressure.

As discussed herein, the drug reservoir and/or drug delivery ampoule may include various vents and/or vapor barriers to facilitate venting, etc. With reference to **FIGS. 9A****-9C,** an exemplary reservoir or ampoule is shown which is configured so as to be insensitive to pressure differentials that may occur when the user travels from sea level to sub-sea levels and at high altitudes, e.g., while traveling in an airplane where pressure differentials may be as great as 4 psi. As shown, **FIG. 9A** shows a perspective view of an exemplary ampoule 900. **FIGS 9B** and **9C** show exploded view of ampoule 900 from perspective top and bottom views. With reference to **FIGS. 9B** and **9C****,** the ampoule 900 generally includes a top cover 901 and a bottom cover 902. The ampoule 900 may be configured to include one or more superhydrophobic filter(s) 904 covering one or more vents 906, and the fluid reservoir housing may include a spiral channel (or similarly shaped) vapor barrier 905, which provides for free exchange of air into and out of the fluid reservoir, while blocking moisture or fluids from passing into the reservoir, thereby reducing or preventing fluid leakage or deposition on aperture plate surfaces. If desired, one or more O-rings 903, or similar sealing mechanism, may be used to form a seal between the top cover 901 and the bottom cover 902 in connection with the vapor barrier 905. Without intending to be limited, the superhydrophobic filter and vent may generally allow for the venting of air and equilibration of air pressure within the fluid reservoir, while maintaining a sterile environment within the fluid reservoir. The spiral channel vapor barrier will generally prevent the transfer of moisture to and from the fluid reservoir (e.g., through the vent opening).

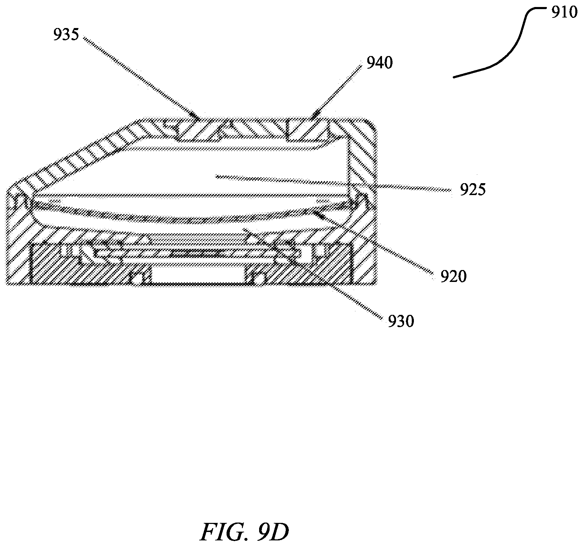

In another embodiment, shown in **FIG. 9D****,** a cross-section of an exemplary small volume drug ampoule 910 is illustrated. As shown, the small volume drug ampoule 910 includes a membrane 920, which separates the reservoir into two volumes, a first background pressure fluid volume 925, and a second drug fluid volume 930. The small volume ampoule may also include an air exchange vent (e.g., a superhydrophobic filter) 935, and an option fill port 940. Any suitable size and shape configuration of reservoir may be used. By way of non-limiting example, for 20 uL dose on a 5 mm ejector, a small volume ampoule may be sized and shaped so as to be 5 mm diameter by 1 mm high well.

In accordance with aspects, the handheld nebulizer devices of the disclosure may include an air inlet flow element (see, e.g., **FIGS. 10A****-10C** and **12A-19D)** which may be positioned in the airflow at the airflow entrance of the device and configured to facilitate non-turbulent (i.e., laminar and/or transitional) airflow across the exit side of aperture plate and to provide sufficient airflow to ensure that the ejected stream of droplets flows through the handheld nebulizer device during use. In some embodiments, the air inlet flow element may be positioned within the mouthpiece. Again, the mouthpiece may include an exhalation valve. Aspects of the present embodiment further allows customizing the internal pressure resistance of the particle delivery device by allowing the placement of laminar flow elements having openings of different sizes and varying configurations to selectively increase or decrease internal pressure resistance, as will be explained in further detail herein.

In accordance with certain embodiments of the handheld nebulizer device of the disclosure, the device may include an air inlet flow element may be positioned in the airflow at the airflow entrance of the device and configured to facilitate non-turbulent (i.e., laminar and/or transitional) airflow across the exit side of aperture plate and to provide sufficient airflow to ensure that the ejected stream of droplets flows through the handheld nebulizer device during use. In some embodiments, the air inlet flow element may be positioned within the mouthpiece (which may include an exhalation valve). In addition, the air inlet flow element allows for customization of internal device pressure resistance by designing openings of different sizes and varying configurations to selectively increase or decrease internal pressure resistance.

As will be described in further detail herein, the air inlet flow element may be positioned behind the exit side of the aperture plate along the direction of airflow, or in-line or in front of the exit side of the aperture plate along the direction of airflow. In certain embodiments, the air inlet flow element comprises one or more openings formed there through and configured to increase or decrease internal pressure resistance within the handheld nebulizer device during use. For instance, the air inlet flow element comprises an array of one or openings. In the embodiments, the air inlet flow element comprises one or more baffles, e.g., wherein the one or more baffles comprise one or more airflow openings.

In certain embodiments, the air inlet flow element is designed and configured in order to provide an optimum airway resistance for achieving peak inspirational flows that are required for deep inhalation which promotes delivery of ejected droplets deep into the pulmonary airways. Air inlet flow elements also function to promote non-turbulent flow across the aerosol plume exit port, which also serves to stabilize airflow repeatability, stability and insures an optimal precision in the delivered dose.

Without intending to be limited by theory, in accordance with aspects of the disclosure, the size, number, shape and orientation of flow restrictions (e.g., openings, holes, flow blocks, etc.) in the air inlet flow element of the disclosure may be configured to provide a desired pressure drop within the handheld nebulizer device. In certain embodiments, it may be generally desirable to provide a pressure drop that is not so large as to strongly affect a user's breathing or perception of breathing.

In certain implementations, the use of air inlet flow elements having differently configured, sized, and shaped flow restrictions (e.g., openings, holes, flow blocks, etc.), or the use of adjustable apertures may be required in order to accommodate the differences among the lungs and associated inspiratory flow rates of young and old, small and large, and various pulmonary disease states. For example, if the aperture is adjustable by the patient (perhaps by having a slotted ring that can be rotated), then a method may be provided to read the aperture hole setting and lock that position to avoid inadvertent changes of the aperture hole size, hence the flow measurement. Although pressure sensing is an accurate method for flow measurement, other embodiments may use, e.g., hot wires or thermistor types of flow rate measurement methods which lose heat at a rate proportional to flow rate, moving blades (turbine flow meter technology) or by using a spring-loaded plate, without limitation of example.

For instance, **FIGS. 10A****-10C** illustrate certain exemplary air inlet flow elements of the disclosure. **FIGS. 10A****-10C** also illustrate the position of pressure sensors, the mouthpiece, and air channels for reference pressure sensing. However, the disclosure is not so limited, and other configurations including those described herein are contemplated as within the scope of the disclosure. While not being so limited, the air inlet flow elements of **FIGS. 10A****-10C** are particularly suitable for use with the handheld nebulizer devices of **FIGS. 1A****-1B.**

More particularly, **FIG. 10A** illustrates a cross-section of a partial handheld nebulizer device 1000 of the disclosure including a mouthpiece cover 1001, a mouthpiece 1002 (which may include an exhalation valve, not shown), a drug delivery ampoule 1003 comprising a drug reservoir 1004 and an ejector mechanism 1005. As illustrated, the handheld nebulizer device includes an air inlet flow element 1006 having an array of holes 1006a at the air entrance of the mouthpiece 1002. Also shown is a pressure sensor port 1007, which may be used to sense a change in pressure within the mouthpiece. With reference to **FIG. 10B****,** a front view of the device 1000 is illustrated, wherein a second pressure sensor port 1008 is shown to provide for sensing of a reference or ambient pressure.

**FIG. 10C** illustrates a partial exploded view including mouthpiece 1002 and inner housing 1011. As shown, mouthpiece 1002 includes air intake flow element 1006 with an array of holes 1006a, and pressure sensor port 1007. Further, mouthpiece 1002 may include an ejection port 1114 positioned, e.g., on the top surface of the mouthpiece so as to align with the ejector mechanism to allow for ejection of the stream of droplets into the airflow of the device during use. Other sensor ports 1115 may be positioned as desired along the mouthpiece to allow for desired sensor function, e.g., spray detection. The mouthpiece may also include positioning baffle 1116 that interfaces with the base unit upon insertion. Although not shown, as described herein, the mouthpiece may include an exhalation valve as illustrated in **FIGS. 1C** and **1D****.** Inner housing 1011 includes pressure sensor board 1009 and outside channel 1010 for facilitating sensing of reference or ambient pressure. The inner housing further includes a first pressure sensing port 1112 to facilitate sensing of pressure changes within the device (e.g., within the mouthpiece or housing), and a second pressure sensing port 1113 to facilitate sensing of reference or ambient pressure.

In this regard, **FIG. 11A** illustrates differential pressure as a function of flow rates through exemplary air inlet flow elements similar to that of **FIGS. 10A****-10C** as a function of number of holes (29 holes, 23 holes, 17 holes). Referring to **FIG. 11B****,** the flow rate verses differential pressure as a function of hole size is shown to have a liner relationship, when flow rate is plotted as a function of the square root of differential pressure. The number of holes is held constant at 17 holes. These data provide a manner to select a design for an air inlet flow element to provide a desired pressure resistance, as well as provide a model for the relationship between flow rate and differential pressure, as measured in an exemplary droplet delivery device.

### Inspiratory Flow Rate (SLM) = C(SqRt) (Pressure(Pa))

| **Element #** | **Hole Size (mm) (17 holes)** | **Pressure at 10 slm (Pa)** | **Flow at 1000 Pa** | **Equation Constant (C)** |
|---|---|---|---|---|
| 0 | 1.9 | 6 | 149.56 | 4.73 |
| 1 | 2.4 | 2.1 | 169.48 | 5.36 |
| 2 | 2.7 | 1.7 | 203.16 | 6.43 |
| 3 | 3 | 1.3 | 274.46 | 8.68 |

A particular non-limiting exemplary air inlet flow element may 29 holes, each 1.9 mm in diameter. However, the disclosure is not so limited. For example, the air inlet flow element may have hole diameters ranging from, e.g., 0.1 mm in diameter to diameters equal to the cross sectional diameter of the air inlet tube (e.g., 0.5 mm, 1 mm, 1.5 mm, 2 mm, 2.5 mm, 3 mm, 3.5 mm, 4 mm, 4.5 mm, 5 mm, 5.5 mm, 6 mm, 6.5 mm, etc.), and number of holes may range from 1 to the number of holes, for example, to achieve the desire air flow resistance, e.g., 2, 3, 4, 5, 6, 7, 8, 9, 10, 15, 20, 25, 29, 30, 60, 90, 100, 150, etc.

**FIGS. 12A****-19D** illustrate alternative embodiments of air inlet flow elements of the disclosure. **FIGS. 12A****-19D** also illustrate exemplary positioning of air inlet flow elements within the airflow of a device, within the mouthpiece, as well as the interfacing of a mouthpiece including an air inlet flow element to an drug delivery ampoule.

**FIG. 12A** shows an exemplary drug delivery ampoule with a mouthpiece interfaced at the airflow exit side of the device. The mouthpiece includes two airflow entrances on the sides, but no internal air inlet flow elements to provide resistance to airflow. **FIG. 12B** shows a front cross-section and **12C** shows a side cross-section, with **FIG. 12D** showing the same views with exemplary dimensions. **FIGS. 13A** and **14A** show similarly configured mouthpieces with two airflow entrances on the sides, but no internal air inlet flow elements to provide resistance to airflow. Again, **FIGS. 13B** and **14B** show a front cross-section and 13C and 14C show a side cross-section, with **FIGS. 13D** and **14D** showing the same views with exemplary dimensions to illustrate the differences in configurations between the embodiments. For instance, the embodiment of **FIG. 12** has openings that are 6.6 mm long and 2 mm high, the embodiment of **FIG. 13** has openings that are 7.9 mm long and 2.5 mm high, and the embodiment of **FIG. 14** has openings that are 8.1 mm long and 3 mm high. Of course, the disclosure is not limited to these specific dimensions, and varied dimensions and numbers of air inflow openings are envisions as within the scope of the disclosure. Again, although not shown, as described herein, the mouthpiece of **FIGS. 12A****-12D, 13A-13D,** and **14A-14D** may include an exhalation valve as illustrated in **FIGS. 1C** and **1D****.**

**FIG. 15A** shows an exemplary drug delivery ampoule with a mouthpiece interfaced at the airflow exit side of the device. The mouthpiece includes two airflow entrances on the exterior sides of the mouthpiece, and two interior baffles with additional airflow entrances to provide resistance and modeling of airflow. **FIG. 15B** shows a front cross-section and 15C shows a side cross-section, with **FIG. 15D** showing the same views with exemplary dimensions. **FIG. 16A** shows a similarly configured mouthpiece that includes two airflow entrances on the exterior sides of the mouthpiece, and two interior baffles with additional airflow entrances to provide resistance and modeling of airflow. However, the interior baffles of **FIG. 16A** are larger (10 mm in height) than that of **FIG. 15A** (5 mm in height). **FIG. 16B** shows a front cross-section and **16C** shows a side cross-section, with **FIG. 16D** showing the same views with exemplary dimensions. Again, although not shown, as described herein, the mouthpiece of **FIGS. 15A****-15D** and **16A-16D** may include an exhalation valve as illustrated in **FIGS. 1C** and **1D****.**

**FIG. 17A** shows an exemplary drug delivery ampoule with a mouthpiece interfaced at the airflow exit side of the device. The mouthpiece includes two airflow entrances on the exterior sides of the mouthpiece, and a substantially concentric baffle (two arcs that form a circle with the top and bottom of the mouthpiece) with two additional airflow entrances to provide resistance and modeling of airflow. **FIG. 17B** shows a front cross-section and **17C** shows a side cross-section, with **FIG. 17D** showing the same views with exemplary dimensions. **FIG. 18A** shows a similarly configured mouthpiece with a substantially concentric interior baffle, but the interior baffle includes four airflow entrances to provide resistance and modeling of airflow. **FIG. 18B** shows a front cross-section and 18C shows a side cross-section, with **FIG. 18D** showing the same views with exemplary dimensions. Again, although not shown, as described herein, the mouthpiece of **FIGS. 17A-****17D** and **18A-18D** may include an exhalation valve as illustrated in **FIGS. 1C** and **1D****.**

**FIG. 19A** shows an exemplary drug delivery ampoule with a mouthpiece interfaced at the airflow exit side of the device. The mouthpiece includes two airflow entrances on the exterior sides of the mouthpiece, and a substantially concentric baffle with two additional airflow entrances to provide resistance and modeling of airflow. In addition, the interior area of the mouthpiece between the concentric baffle and the wall of the mouthpiece includes an array element positioned above the airflow entrances to provide additional resistance and modeling to airflow. The array element is positioned in a parallel arrangement with the direction of airflow. Again, **FIG. 19B** shows a front cross-section and **19C** shows a side cross-section, with **FIG. 19D** showing the same views with exemplary dimensions. Although not shown, as described herein, the mouthpiece of **FIGS. 19A****-19D** may include an exhalation valve as illustrated in **FIGS. 1C** and **1D****.**

In accordance with the disclosure, it has been found that the presence of inner air inlet flow elements generally improve spray efficiency for exemplary fluid solutions (deionized water and albuterol solution. For instance, as shown in **FIG. 20**, at 30 SLM , inner air inlet flow elements increase spray efficiency from 47% to 66%, and orienting interior airflow entrances away from ejection streams improves spray efficiency to 80% or more. The mouthpiece and drug reservoir are a single unit and can be weighted before ejection (W1), after ejection (W2) and after drying (W3) the mouthpiece to measure the percentage of ejected drug that leaves the mouthpiece for delivery to a user. Spray efficiency = (W1-W2)/(W1-W3)

In certain aspects of the disclosure, the in-line device may be configured to protect the surface of the aperture plate, to minimize evaporation losses, and to minimize contamination while the device is closed and not in use. For instance, as described herein, when the reservoir/ampoule is in the closed position, the surface of the aperture plate of the ejector mechanism may be closed/sealed against the housing or the mouthpiece cover. However, in certain embodiments, when the reservoir/ampoule includes an O-ring or gasket to facilitate the seal of the surface of the aperture plate of the ejector mechanism, the sliding of the reservoir/ampoule between the open and closed position may, in certain aspects, create friction which needs to be overcome by a compression spring during opening and closing.

In one embodiment, friction between the ampoule O-ring and the device housing may be reduced by applying a compressive force between the ampoule and the device housing in the last few millimeters as the ampoule is closed. Thus, higher friction is limited to the first few millimeters during opening, when the compression spring is providing the highest force; and during the last few millimeters of closing when the ampoule door is almost closed and force on the door is easiest for the user to apply. Force applied as the door is almost closed also creates minimal reaction forces at the door's hinge, improving robustness of the device. Applying pressure to the O-ring over a shorter distance also reduces wear on the O-ring (or gasket).

Without being limited, in certain embodiments, applying a compressive sealing force during the last few millimeters of ampoule motion to the closed position can be accomplished by utilizing a ramp on either the ampoule or device side of the ampoule track which engages a budge on the opposite face (device for ampoule or ampoule for device) as the ampoule approaches the closed position. This can also be a pair of ramps which engage as the ampoule approaches the closed position. In certain aspects, the point(s) of contact between the ampoule and device should be in alignment with the center of pressure of the O-ring to create a uniform sealing pressure. Note that to achieve enough compression for good sealing, the total vertical motion created by the ramp only needs to be in the range of 0.1 mm.

Alternatively to a sealing force generated by a fixed movement of the ampoule towards the device, a flexible compressive element can apply a downward force the rises as the ampoule approaches the closed position. By way of non-limiting example, this could be the ramp intersecting a flexible, rubber-like, material or a metallic or plastic spring, including a cantilever (leaf) spring that the ramp encounters as it arrives at the closed position of the ampule.

The compressive force applied to the O-ring does not have to be large, but sufficient for the compliant O-ring to seal against the surface roughness of the device surface. In certain embodiments, a more compliant material will require less compressive force to seal. Similarly, the O-ring can be made from a slippery material such as teflon-coated or teflon-encapsulated material to reduce the sliding friction of the ampule. Similarly, sealing may be done by a lip seal at the face.

**FIGS. 21A****-21C** illustrate exemplary embodiments showing a ramp structure on the ampoule lip that presses the ampoule down and compresses the O-ring while in the "closed" position. Note, as illustrated the size of the ramp is greatly exaggerated. In one embodiment, the ramp may be about 0.1 to 0.2 mm high. **FIG. 21A** shows an end view showing ampule with lips that are engaged in track that is part of body of device. **FIG. 21B** shows how an ampoule moves from closed to open position. Mouthpiece and user to the right. **FIG. 21C** illustrates a side view of an ampoule in track with a ramp on a lip to force a aperture plate seal, showing a closed and open position.

In other embodiments, the surface of the aperture plate may be protected by the mouthpiece cover. For instance, as shown in **FIG. 21D****,** mouthpiece cover 2100 may include aperture plate plug 2102 that is specifically sized and shaped so as to form a mating seal against the surface of the aperture plate 2104 when the cover is closed. In certain embodiments, the aperture plate plug 2102 may have a stepped shape such that the plug forms a seal against the surface of the housing around the aperture plate without putting direct pressure on the surface of the aperture plate.

In certain embodiments, as illustrated herein, the reservoir/cartridge module may include components that may carry information read by the housing electronics including key parameters such as ejector mechanism functionality, drug identification, and information pertaining to patient dosing intervals. Some information may be added to the module at the factory, and some may be added at the pharmacy. In certain embodiments, information placed by the factory may be protected from modification by the pharmacy. The module information may be carried as a printed barcode or physical barcode encoded into the module geometry (such as light transmitting holes on a flange which are read by sensors on the housing). Information may also be carried by a programmable or non-programmable microchip on the module which communicates to the electronics in the housing.

By way of example, module programming at the factory or pharmacy may include a drug code which may be read by the device, communicated via Bluetooth^{®} to an associated user smartphone and then verified as correct for the user. In the event a user inserts an incorrect, generic, damaged, etc., module into the device, the smartphone might be prompted to lock out operation of the device, thus providing a measure of user safety and security not possible with passive inhaler devices. In other embodiments, the device electronics can restrict use to a limited time period (perhaps a day, or weeks or months) to avoid issues related to drug aging or build-up of contamination or particulates within the device housing.

The handheld nebulizer device may further include various sensors and detectors to facilitate device activation, spray verification, patient compliance, diagnostic mechanisms, or as part of a larger network for data storage, big data analytics and for interacting and interconnected devices used for subject care and treatment, as described further herein. Further, the housing may include an LED assembly on a surface thereof to indicate various status notifications, e.g., ON/READY, ERROR, etc.

The airflow exit of the housing of the handheld nebulizer device through which the ejected plume of droplets exit as they are inhaled into a subject's airways, may be configured and have, without limitation, a cross sectional shape of a circle, oval, rectangular, hexagonal or other shape, while the shape of the length of the tube, again without limitation, may be straight, curved or have a Venturi-type shape.

In another embodiment (not shown), a mini fan or centrifugal blower may be located at the air inlet side of the laminar flow element or internally of the housing within the airsteam. The mini fan generally may provide additional airflow and pressure to the output of the plume. For patients with low pulmonary output, this additional airplume may ensure that the plume of droplets is pushed through the device into the patient's airway. In certain implementations, this additional source of airflow ensures that the plume exit port is swept clean of the droplets and also provides mechanism for spreading the particle plume into an airflow which creates greater separation between droplets. The airflow provided by the mini fan may also act as a carrier gas, ensuring adequate dose dilution and delivery.

In other embodiments, the internal pressure resistance of the handheld nebulizer device may be customized to an individual user or user group by modifying the mouthpiece tube design to include various configurations of air aperture grids or openings, thereby increasing or decreasing resistance to airflow through the device as the user inhales. For instance, different air entrance aperture sizes and numbers may be used to achieve different resistance values, and thereby different internal device pressure values. This feature provides a mechanism to easily and quickly adapt and customize the airway resistance of the particle delivery device to the individual patient's state of health or condition.

In another aspect of the disclosure, in certain embodiments, the handheld nebulizer devices provide for various automation, monitoring and diagnostic functions. By way of example, as described above, device actuation may be provided by way of automatic subject breath actuation. Further, in certain embodiments, the device may provide automatic spray verification, to ensure that the device has generated the proper particle generation and provided to proper dosing to the subject. In this regard, the particle delivery device may be provided with one or more sensors to facilitate such functionality.

For instance, an airflow sensor located in the mouthpiece may measure inspiratory and expiratory flow rates. This sensor is placed so that it does not interfere with drug delivery or become a site for collection of residue or promote bacterial growth or contamination. A differential (or gage) pressure sensor downplume of a flow restrictor (e.g., air inlet flow element) measures airflow based upon the pressure differential between the inside of the mouthpiece relative to the outside air pressure. During inhalation (inspiratory flow) the mouthpiece pressure will be lower than the ambient pressure and during exhalation (expiratory flow) the mouthpiece pressure will be greater than the ambient pressure. The magnitude of the pressure differential during an inspiratory cycle is a measure of the magnitude of airflow and airway resistance at the air inlet end of the delivery tube.

Again, a Bluetooth^{®} communication module or similar wireless communication module may be provided in order to link the handheld nebulizer device to a smartphone or other similar smart devices (not shown). Bluetooth^{®} connectivity facilitates implementation of various software or App's which may provide and facilitate patient training on the use of the device. A major obstacle to effective inhaler drug therapy has been either poor patient adherence to prescribed aerosol therapy or errors in the use of an inhaler device. By providing a real time display on the smartphone screen of a plot of the patient's inspiratory cycle, (flow rate versus time) and total volume, the patient may be challenged to reach a goal of total inspiratory volume that was previously established and recorded on the smartphone during a training session in a doctor's office. Bluetooth^{®} connectivity further facilitates patient adherence to prescribed drug therapy and promotes compliance by providing a means of storing and archiving compliance information, or diagnostic data (either on the smartphone or cloud or other large network of data storage) that may be used for patient care and treatment.

More specifically, in certain embodiments, the handheld nebulizer device may provide automatic spray verification via LED and photodetector mechanisms. For instance, an infra-red transmitter (e.g., IR LED, or UV LED < 280 nm LED), and infra-red or UV (UV with <280nm cutoff) photodetector may be mounted along the droplet ejection side of the device to transmit an infra-red or UV beam or pulse, which detects the plume of droplets and thereby may be used for spray detection and verification. The IR or UV signal interacts with the aerosol plume and can be used to verify that a plume of droplets has been ejected as well as provide a measure of the corresponding ejected dose of medicament. Examples include but not limited to, infrared 850 nm emitters with narrow viewing angles of either, 8, 10 and 12-degrees, (MTE2087 series) or 275 nm UV LED with a GaN photodetector for aerosol plume verification in the solar blind region of the spectra. Alternatively in some applications, the sub 280 nm LEDs (e.g. 260 nm LEDs) can be used to disinfect the spacer tube 128.

By way of example, the concentration of a medicament in the ejected fluid may be made, according to Beer's Law Equation (Absorbance = e L c), where, e is the molar absorptivity coefficient (or molar extinction coefficient) which is a constant that is associated with a specific compound or formulation, L is the path length or distance between LED emitter and photodetector, and c is the concentration of the solution. This implementation provides a measure of drug concentration and can be used for verification and a means and way to monitoring patient compliance as well as to detect the successful delivery of medication.

In another embodiment, spray verification and dose verification can be monitored by measuring the transmission of 850 nM to 950 nM light across the spray in a region where the droplets are not variably diluted with different inhalation flow rates. The average and alternating signals from the detector may be measured to calibrate and confirm the optical path (average signal) and detect the spray (alternating signal). In practice, the alternating signal can be measured by a 100 Hz low-pass filter between the detector and analog converter, sampling the signal 100 to 500 times a second, calculating the average and the range (maximum minus minimum) over 100 mS periods, and comparing these values to preset values to confirm proper operation and whether there was spray or not.

This method has the strong advantages of: low power consumption (less than 1 ma to the emitter); unaffected by stray light (visible light blocking on the detector); relatively resistant to digital noise or the 100 kHz piezo drive by the 100 Hz low-pass filter; the average signal level can be used to adjust the optical path for attenuation caused by drug deposits on the LED or detector; and simple hardware with a positive signal that is robustly measured.

This system also allows simple regulation of the optical signal strength by increasing power to the emitter should the average signal level decrease. Practically, this means using pulse width modulation of emitter current to regulate average emitter power. The pulses should be at a high rate, e.g., 100 kHz, so that this noise can be removed by the 100 Hz low pass filter. Nominal operation might use a 10% duty cycle of 10 mA to achieve and average current of 1 mA. This system would have the ability to increase the average current to 10 mA and correct for up to a factor of 10 attenuation by drug deposits.

In operation with the 950 nM emitter and detector having angles of +-20 degrees and spaced 10 mm apart. With 0.5 mA emitter power, a 10K collector resistor and 100 Hz low-pass filter, the average signal output is 2 volts and the peak to peak value of the alternating component is 4 mV without spray and 40 mV during spray. Without intending to be limited, in practice, there may be a transient large peak to peak value when the spray begins and ends as the bulk attenuation causes a large shift. The resistor sizing here is for continuous running of the emitter and not PWM.

While the disclosure has been described with reference to exemplary embodiments, it will be understood by those skilled in the art that various changes may be made and equivalents may be substituted for elements thereof without departing from the scope of the disclosure. In addition, many modifications may be made to adapt a particular situation or material to the teachings without departing from the essential scope thereof. Therefore, it is intended that the disclosure not be limited to the particular embodiment disclosed, but that the disclosure will include all embodiments falling within the scope of the appended claims.

## Claims

1. A breath actuated, handheld nebulizer device (400) for delivering a fluid as an ejected stream of droplets to the pulmonary system of a subject, the device (400) comprising:
a housing;
a mouthpiece (108, 408) positioned at an airflow exit of the device (400), the mouthpiece (108, 408)including an exhalation valve (108a);
an air inlet flow element (1006) positioned in the airflow at an airflow entrance of the device (400);
a fluid reservoir (435) disposed in or in fluid communication with the housing for receiving a volume of fluid;
an ejector mechanism (434) in fluid communication with the reservoir and configured to generate the ejected stream of droplets;
at least one differential pressure sensor (204) positioned within the housing, the at least one differential pressure sensor (204) configured to activate the ejector mechanism (434) upon sensing a pre-determined pressure change within the mouthpiece (108, 408) to thereby generate the ejected stream of droplets;
the ejector mechanism (434) comprising a piezoelectric actuator and an aperture plate, the aperture plate having a plurality of openings formed through its thickness and the piezoelectric actuator operable to oscillate the aperture plate at a frequency to thereby generate the ejected stream of droplets;
wherein the housing, air inlet flow element (1006), and mouthpiece (108, 408) are configured to facilitate non-turbulent airflow across an exit side of the aperture plate and to provide sufficient airflow through the housing during use; and
wherein the ejector mechanism is configured to generate the ejected stream of droplets wherein at least about 50% of the droplets have an average ejected droplet diameter of less than about 6 microns, such that at least about 50% of the mass of the ejected stream of droplets is delivered in a respirable range to the pulmonary system of the subject during use.

2. The handheld nebulizer device of claim 1, wherein the exhalation valve is a one-way valve configured to open during use to release exhalation pressure.

3. The handheld nebulizer device of claim 1, wherein the housing and ejector mechanism are oriented such that the exit side of the aperture plate is perpendicular to the direction of airflow and the stream of droplets is ejected in parallel to the direction of airflow or such that the exit side of the aperture plate is parallel to the direction of airflow and the stream of droplets is ejected substantially perpendicularly to the direction of airflow such that the ejected stream of droplets is directed through the housing at an approximate 90 degree change of trajectory prior to expulsion from the housing.

4. The handheld nebulizer device (400) of claim 1, wherein the air inlet flow element 1006) is positioned within the mouthpiece (108, 408), wherein optionally the air inlet flow element (1006) is positioned behind the exit side of the aperture plate along the direction of airflow or is positioned in-line or in front of the exit side of the aperture plate along the direction of airflow.

5. The handheld nebulizer device (400) of claim 1, wherein the air inlet flow element (10069) comprises one or more openings formed there through and configured to increase or decrease internal pressure resistance within the handheld nebulizer device during use.

6. The handheld nebulizer device (400) of claim 5, wherein the air inlet flow element (1006) comprises an array of one or more openings.

7. The handheld nebulizer device (400) of claim 5, wherein the air inlet flow element (1006) comprises one or more baffles, optionally wherein the one or more baffles comprise one or more airflow openings.

8. The handheld nebulizer device (400) of claim 1, wherein the aperture plate comprises a domed shape.

9. The handheld nebulizer device (400) of claim 1, wherein the aperture plate is composed of a material selected from the group consisting of poly ether ether ketone (PEEK), polyimide, polyetherimide, polyvinylidine fluoride (PVDF), ultra-high molecular weight polyethylene (UHMWPE), nickel, nickel-cobalt, nickel-palladium, palladium, platinum, metal alloys thereof, and combinations thereof.

10. The handheld nebulizer device (400) of claim 1, wherein one or more of the plurality of openings have different cross-sectional shapes or diameters to thereby provide ejected droplets having different average ejected droplet diameters.

11. The handheld nebulizer device (400) of claim 1, wherein the mouthpiece (108, 408) is removably coupled with the device.

12. The handheld nebulizer device (400) of claim 1, wherein the reservoir (435) is removably coupled with the housing.

13. The handheld nebulizer device (400) of claim 1, wherein the reservoir (435) is coupled to the ejector mechanism (434) to form a combination reservoir/ejector mechanism module, and the combination reservoir/ejector mechanism module is removably coupled with the housing.

14. The handheld nebulizer device (400) of claim 1, further comprising a wireless communication module.

15. The handheld nebulizer device (400) of claim 1, wherein the device further comprises one or more sensors selected from an infra-red transmitter, a photodetector, an additional pressure sensor, and combinations thereof.

## Patentansprüche

1. Atemaktivierte, tragbare Verneblervorrichtung (400) zum Zuführen eines Fluids als ausgestoßener Strom von Tröpfchen an das pulmonale System eines Individuums, wobei die Vorrichtung (400) Folgendes umfasst:
ein Gehäuse;
ein Mundstück (108, 408), das an einem Luftströmungsausgang der Vorrichtung (400) angeordnet ist, wobei das Mundstück (108, 408) ein Ausatmungsventil (108a) umfasst;
ein Lufteinlassströmungselement (1006), das in der Luftströmung an einem Luftströmungseingang der Vorrichtung (400) angeordnet ist;
ein Fluidreservoir (435), das in dem Gehäuse oder in Fluidkommunikation damit zum Aufnehmen eines Fluidvolumens angeordnet ist;
einen Ausstoßmechanismus (434) in Fluidkommunikation mit dem Reservoir, und das ausgelegt ist, um den ausgestoßenen Tröpfchenstrom zu erzeugen;
zumindest einen Differenzdrucksensor (204), der innerhalb des Gehäuses angeordnet ist, wobei der zumindest eine Differenzdrucksensor (204) ausgelegt ist, um den Ausstoßmechanismus (434) bei Erkennen einer vorbestimmten Druckänderung innerhalb des Mundstücks (108, 408) zu aktivieren, um dadurch den ausgestoßenen Tröpfchenstrom zu erzeugen;
wobei der Ausstoßmechanismus (434) ein piezoelektrisches Aktuatorelement und eine Öffnungsplatte umfasst, wobei die Öffnungsplatte eine Vielzahl von Öffnungen aufweist, die durch deren Dicke hindurch ausgebildet sind, und wobei das piezoelektrische Aktuatorelement betreibbar ist, um die Öffnungsplatte mit einer Frequenz zu schwingen, um dadurch den ausgestoßenen Tröpfchenstrom zu erzeugen;
wobei das Gehäuse, das Lufteinlassströmungselement (1006) und das Mundstück (108, 408) ausgelegt sind, um eine nichtturbulente Luftströmung über eine Ausgangsseite der Öffnungsplatte zu ermöglichen und um während der Verwendung eine ausreichende Luftströmung durch das Gehäuse bereitzustellen; und
wobei der Ausstoßmechanismus ausgelegt ist, um den ausgestoßenen Tröpfchenstrom zu erzeugen, wobei zumindest etwa 50 % der Tröpfchen einen durchschnittlichen ausgestoßenen Tröpfchendurchmesser von weniger als etwa 6 µm aufweisen, sodass während der Verwendung zumindest etwa 50 % der Masse des ausgestoßenen Tröpfchenstroms in einem atembaren Bereich an das pulmonale System des Individuums zugeführt werden.

2. Tragbare Verneblervorrichtung nach Anspruch 1, wobei das Ausatmungsventil ein Einwegventil ist, das ausgelegt ist, um sich während der Verwendung zu öffnen, um Ausatmungsdruck abzulassen.

3. Tragbare Verneblervorrichtung nach Anspruch 1, wobei das Gehäuse und der Ausstoßmechanismus so ausgerichtet sind, dass die Ausgangsseite der Öffnungsplatte normal auf die Richtung des Luftstroms steht und der Tröpfchenstrom parallel zur Richtung des Luftstroms oder so ausgestoßen wird, dass die Ausgangsseite der Öffnungsplatte parallel zur Richtung des Luftstroms ist und der Tröpfchenstrom im Wesentlichen normal auf die Richtung des Luftstroms ausgestoßen wird, sodass der ausgestoßene Tröpfchenstrom durch das Gehäuse in einer Trajektorienänderung von etwa 90° vor Ausstoßen aus dem Gehäuse geleitet wird.

4. Tragbare Verneblervorrichtung (400) nach Anspruch 1, wobei das Lufteinlassströmungselement (1006) innerhalb des Mundstücks (108, 408) angeordnet ist, wobei das Lufteinlassströmungselement (1006) gegebenenfalls hinter der Ausgangsseite der Öffnungsplatte entlang der Richtung des Luftstroms angeordnet ist oder in Serie mit oder vor der Ausgangsseite der Öffnungsplatte entlang der Richtung des Luftstroms angeordnet ist.

5. Tragbare Verneblervorrichtung (400) nach Anspruch 1, wobei das Lufteinlassströmungselement (1006) eine oder mehrere Öffnungen umfasst, die durch dieses hindurch ausgebildet und ausgelegt sind, um den inneren Druckwiderstand innerhalb der tragbaren Verneblervorrichtung während der Verwendung zu verringern.

6. Tragbare Verneblervorrichtung (400) nach Anspruch 5, wobei das Lufteinlassströmungselement (1006) eine Anordnung einer oder mehrerer Öffnungen umfasst.

7. Tragbare Verneblervorrichtung (400) nach Anspruch 5, wobei das Lufteinlassströmungselement (1006) ein oder mehrere Leitelemente umfasst, wobei das eine oder die mehreren Leitelemente gegebenenfalls eine oder mehrere Luftströmungsöffnungen umfassen.

8. Tragbare Verneblervorrichtung (400) nach Anspruch 1, wobei die Öffnungsplatte eine kuppelartige Form umfasst.

9. Tragbare Verneblervorrichtung (400) nach Anspruch 1, wobei die Öffnungsplatte aus einem Material besteht, das aus der aus Polyetheretherketon (PEEK), Polyimid, Polyetherimid, Polyvinylidienfluorid (PVDF), Polyethylen mit ultrahohem Molekulargewicht (UHMWPE), Nickel, Nickel-Cobalt, Nickel-Palladium, Palladium, Platin, Metalllegierungen davon und Kombinationen daraus bestehenden Gruppe ausgewählt ist.

10. Tragbare Verneblervorrichtung (400) nach Anspruch 1, wobei eine oder mehrere aus der Vielzahl von Öffnungen verschiedene Querschnittsformen oder -durchmesser aufweisen, um dadurch ausgestoßene Tröpfchen mit verschiedenen durchschnittlichen ausgestoßenen Tröpfchendurchmessern bereitzustellen.

11. Tragbare Verneblervorrichtung (400) nach Anspruch 1, wobei das Mundstück (108, 408) lösbar mit der Vorrichtung gekoppelt ist.

12. Tragbare Verneblervorrichtung (400) nach Anspruch 1, wobei das Reservoir (435) lösbar mit dem Gehäuse gekoppelt ist.

13. Tragbare Verneblervorrichtung (400) nach Anspruch 1, wobei das Reservoir (435) mit dem Ausstoßmechanismus (434) gekoppelt ist, um ein Reservoir/Ausstoßmechanismus-Kombinationsmodul auszubilden, und wobei das Reservoir/Ausstoßmechanismus-Kombinationsmodul lösbar mit dem Gehäuse gekoppelt ist.

14. Tragbare Verneblervorrichtung (400) nach Anspruch 1, die ferner ein Drahtloskommunikationsmodul umfasst.

15. Tragbare Verneblervorrichtung (400) nach Anspruch 1, wobei die Vorrichtung ferner einen oder mehrere Sensoren umfasst, die aus einem Infrarotsender, einem Photodetektor, einem zusätzlichen Drucksensor und Kombinationen daraus ausgewählt sind.

## Revendications

1. Dispositif nébuliseur portatif, actionné par la respiration (400) pour administrer un fluide sous la forme d'un flux de gouttelettes éjecté vers le système pulmonaire d'un sujet, le dispositif (400) comprenant :
un logement ;
un embout buccal (108, 408) positionné au niveau d'une sortie d'écoulement d'air du dispositif (400), l'embout buccal (108, 408)comprenant une valve d'expiration (108a) ;
un élément d'écoulement d'entrée d'air (1006) positionné dans l'écoulement d'air au niveau d'une entrée d'écoulement d'air du dispositif (400) ;
un réservoir de fluide (435) disposé à l'intérieur du logement, ou en communication fluidique avec celui-ci, pour recevoir un volume de fluide ;
un mécanisme éjecteur (434) en communication fluidique avec le réservoir et configuré pour générer le flux de gouttelettes éjecté ;
au moins un capteur de pression différentielle (204) positionné dans le boîtier, le au moins un capteur de pression différentielle (204) étant configuré pour activer le mécanisme éjecteur (434) lors de la détection d'un changement de pression prédéterminé dans l'embout buccal (108, 408) pour générer ainsi le flux de gouttelettes éjecté ;
le mécanisme éjecteur (434) comprenant un actionneur piézoélectrique et une plaque à ouvertures, la plaque à ouvertures présentant une pluralité d'ouvertures formées à travers son épaisseur, et l'actionneur piézoélectrique pouvant fonctionner pour faire osciller la plaque à ouvertures à une fréquence pour générer ainsi le flux de gouttelettes éjecté ;
dans lequel le logement, l'élément d'écoulement d'entrée d'air (1006) et l'embout buccal (108, 408) sont configurés pour faciliter un écoulement d'air non turbulent à travers un côté de sortie de la plaque à ouvertures et pour fournir un écoulement d'air suffisant à travers le logement en utilisation ; et
dans lequel le mécanisme éjecteur est configuré pour générer le flux de gouttelettes éjecté dans lequel au moins environ 50 % des gouttelettes présentent un diamètre moyen de gouttelettes éjectées inférieur à environ 6 microns, de telle sorte qu'au moins environ 50 % de la masse du flux de gouttelettes éjecté sont administrés dans une plage respirable vers le système pulmonaire du sujet en utilisation.

2. Dispositif nébuliseur portatif selon la revendication 1, dans lequel la valve d'expiration est une valve antireflux configurée pour s'ouvrir en utilisation afin de libérer la pression d'expiration.

3. Dispositif nébuliseur portatif selon la revendication 1, dans lequel le logement et le mécanisme éjecteur sont orientés de telle sorte que le côté de sortie de la plaque à ouvertures est perpendiculaire à la direction d'écoulement d'air et le flux de gouttelettes est éjecté parallèlement à la direction d'écoulement d'air ou de telle sorte que le côté de sortie de la plaque à ouvertures est parallèle à la direction d'écoulement d'air et le flux de gouttelettes est éjecté sensiblement perpendiculairement à la direction d'écoulement d'air, de telle sorte que le flux de gouttelettes éjecté est dirigé à travers le logement à un changement de trajectoire d'environ 90 degrés avant d'être expulsé du boîtier.

4. Dispositif nébuliseur portatif (400) selon la revendication 1, dans lequel l'élément d'écoulement d'entrée d'air (1006) est positionné au sein de l'embout buccal (108, 408), dans lequel facultativement l'élément d'écoulement d'entrée d'air (1006) est positionné derrière le côté de sortie de la plaque à ouvertures le long de la direction d'écoulement d'air ou est positionné en ligne ou en face du côté de sortie de la plaque à ouvertures le long de la direction d'écoulement d'air.

5. Dispositif nébuliseur portatif (400) selon la revendication 1, dans lequel l'élément d'écoulement d'entrée d'air (1006) comprend une ou plusieurs ouvertures formées à travers celui-ci et configurées pour augmenter ou diminuer la résistance à la pression interne à l'intérieur du dispositif nébuliseur portatif en utilisation.

6. Dispositif nébuliseur portatif (400) selon la revendication 5, dans lequel l'élément d'écoulement d'entrée d'air (1006) comprend un réseau d'une ou plusieurs ouvertures.

7. Dispositif nébuliseur portatif (400) selon la revendication 5, dans lequel l'élément d'écoulement d'entrée d'air (1006) comprend un ou plusieurs déflecteurs, facultativement dans lequel les un ou plusieurs déflecteurs comprennent une ou plusieurs ouvertures d'écoulement d'air.

8. Dispositif nébuliseur portatif (400) selon la revendication 1, dans lequel la plaque à ouvertures comprend une forme en dôme.

9. Dispositif nébuliseur portatif (400) selon la revendication 1, dans lequel la plaque à ouvertures est composée d'un matériau choisi dans le groupe constitué de polyétheréthercétone (PEEK), polyimide, polyétherimide, fluorure de polyvinylidine (PVDF), polyéthylène à poids moléculaire ultra-élevé (UHMWPE), nickel, nickel-cobalt, nickel-palladium, palladium, platine, d'alliages métalliques de ceux-ci, et de combinaisons de ceux-ci.

10. Dispositif nébuliseur portatif (400) selon la revendication 1, dans lequel une ou plusieurs de la pluralité d'ouvertures présentent des formes ou diamètres de section transversale différents pour fournir ainsi des gouttelettes éjectées présentant des diamètres moyens de gouttelettes éjectées différents.

11. Dispositif nébuliseur portatif (400) selon la revendication 1, dans lequel l'embout buccal (1002, 108) est couplé de manière amovible au dispositif.

12. Dispositif nébuliseur portatif (400) selon la revendication 1, dans lequel le réservoir (435) est couplé de manière amovible au logement.

13. Dispositif nébuliseur portatif (400) selon la revendication 1, dans lequel le réservoir (435) est couplé au mécanisme éjecteur (434) pour former un module de réservoir/mécanisme éjecteur en combinaison, et le module de réservoir/mécanisme éjecteur en combinaison est couplé de manière amovible au logement.

14. Dispositif nébuliseur portatif selon la revendication 1, comprenant en outre un module de communication sans fil.

15. Dispositif nébuliseur portatif (400) selon la revendication 1, dans lequel le dispositif comprend en outre un ou plusieurs capteurs choisis parmi un émetteur infrarouge, un photodétecteur, un capteur de pression supplémentaire, et des combinaisons de ceux-ci.
